(19) Europäisches Patentamt
European Patent Office
Office européen des brevets

(11) Publication number: **0 018 735**
**B1**

(12) # EUROPEAN PATENT SPECIFICATION

(45) Date of publication of patent specification: **27.07.83**

(21) Application number: **80301157.6**

(22) Date of filing: **10.04.80**

(51) Int. Cl.$^3$: **C 07 D 471/04,**
A 61 K 31/44 //(C07D471/04,
221/00)

(54) **1,8-Naphthyridine derivatives, their preparation, pharmaceutical compositions containing them.**

(30) Priority: **18.04.79 US 31255**
**28.02.80 US 125600**

(43) Date of publication of application:
**12.11.80 Bulletin 80/23**

(45) Publication of the grant of the patent:
**27.07.83 Bulletin 83/30**

(84) Designated Contracting States:
**AT BE CH DE FR IT LI LU NL SE**

(56) References cited:
**GB - A - 1 397 869**

(73) Proprietor: **AMERICAN HOME PRODUCTS CORPORATION**
**685, Third Avenue**
**New York, New York 10017 (US)**

(72) Inventor: **Santilli, Arthur Attilio**
**1737, Sue Ellen Drive**
**Havertown Pennsylvania (US)**
Inventor: **Scotese, Anthony Carmen**
**240, Beidler Road**
**King of Prussia Pennsylvania (US)**

(74) Representative: **Wileman, David Francis et al,**
**c/o John Wyeth and Brother Limited**
**Huntercombe Lane South**
**Taplow Maidenhead Berkshire SL6 OPH (GB)**

**EP 0 018 735 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

1,8 Naphthyridine derivatives, their preparation, pharmaceutical compositions containing them

This invention relates to 1,8-naphthyridine derivatives.

More particularly this invention relates to a group of 4-amino or substituted amino-3-substituted-1,2-dihydro-2-oxo-1,8-naphthyridine derivatives which act as gastric anti-secretory agents and may be used in the treatment of peptic ulcer disease.

The anti-ulcer agents of this invention function in their anti-secretory capacity to reduce (1) total gastric volume, (2) hydrogen ion secretion, or (3) hydrogen ion concentration. The reduction of any one of these parameters aids in attenuating the general debilitating influence of a peptic ulcer. The use of compounds exhibiting anti-secretory activity in the curative and/or prophylactic treatment of peptic ulcer disease is an established, beneficial procedure.

U.K. Patent Specification No. 1,397,869 discloses 2-oxo-1,8-naphthyridine derivatives unsubstituted in the 4-position which inhibit bronchial constriction and exhibit hypotensive properties.

Accordingly this invention provides 4-amino and 4-substituted amino-1,8-naphthyridine derivatives of formula

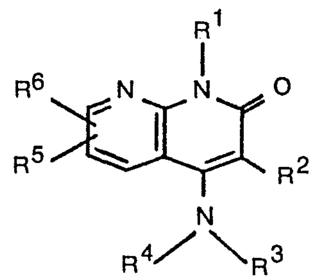

(I)

in which $R^1$ is hydrogen, alkyl of 1 to 6 carbon atoms, cycloalkyl of 5 to 6 carbon atoms, alken-(3,4,5 or 6)-yl of 3 to 6 carbon atoms or alkyn-(3,4,5 or 6)-yl of 3 to 6 carbon atoms; $R^2$ is $-CN$, $-CO_2H$, $-CO_2R^7$ where $R^7$ is alkyl of 1 to 6 carbon atoms, $-CONHNH_2$ or

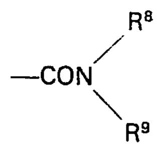

where $R^8$ and $R^9$ are independently hydrogen or alkyl of 1 to 6 carbon atoms; $R^3$ and $R^4$ are, independently, hydrogen, alkyl of 1 to 6 carbon atoms, hydroxyalkyl of 1 to 6 carbon atoms, dialkylaminoalkyl of 4 to 8 carbon atoms, alkanoyl of 2 to 4 carbon atoms, haloalkanoyl of 2 to 4 carbon atoms, or when taken together with the nitrogen atom to which they are attached, $R^3$ and $R^4$ complete a heterocyclic group selected from aziridinyl, azetidinyl, pyrrolidinyl, piperidinyl piperazinyl, 4-lower alkylpiperazinyl, 4-phenylpiperazinyl, 4-[1-ethyl-1,2-dihydro-7-methyl-2-oxo-1,8-naphthyridine-4-yl-3-carboxylic acid ethyl ester]piperazinyl, 4-carb(lower)alkoxypiperazinyl, morpholinyl, thiomorpholinyl or a ring substituted lower alkyl analogue thereof; $R^5$ and $R^6$ are independently hydrogen, alkyl of 1 to 4 carbon atoms, halo, alkylamino of 1 to 4 carbon atoms, or alkoxy of 1 to 6 carbon atoms; or a pharmaceutically acceptable salt thereof.

When either $R^1$ or $R^7$ is alkyl, they preferably have 1 to 4 carbon atoms. Preferably $R^3$ and $R^4$ are both hydrogen or together with the nitrogen atom represent pyrrolidinyl, morpholinyl, piperazinyl or 4-methylpiperazinyl. Preferably $R^5$ is hydrogen and $R^6$ is hydrogen or methyl.

The preferred compounds of the invention from the standpoint of potency as anti-secretory agents are those of the formula:

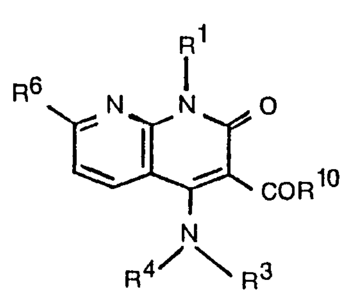

(II)

in which $R^1$ is hydrogen, methyl, ethyl, propyl, allyl, propargyl, cyclohexyl or isobutyl; $R^3$ is hydrogen, alkyl of 1 to 6 carbon atoms or trifluoroacetyl; $R^4$ is hydrogen, alkyl of 1 to 6 carbon atoms, or when

taken with $R^3$ and the nitrogen atom to which they are attached forms a heterocyclic group selected from pyrrolidinyl, morpholinyl, piperazinyl, or 4-methyl-piperazinyl; $R^6$ is hydrogen or methyl; and $R^{10}$ is hydroxy, alkoxy of 1 to 6 carbon atoms or hydrazinyl; or a pharmaceutically acceptable salt thereof.

Those compounds containing a basic amino group in the 4-position, such as the piperazinyl and dialkylaminoalkyl groups, are capable of forming acid addition salts. It is intended throughout this specification and claims to embrace the pharmaceutically acceptable salts of such compounds, which salts are conveniently derived from such acids as hydrochloric, hydrobromic, sulfuric, phosphoric, methane sulfonic, nitric, p-toluene sulfonic, acetic, citric, maleic, succinic acid and the like. In addition, the compounds in their free carboxylic acid form are converted by standard techniques well-known to the chemist into alkali metal (e.g. sodium or potassium), alkaline earth metal (e.g. calcium or magnesium), ammonium or primary, secondary and tertiary alkylamine salts, the latter containing from 1 to 6 carbon atoms in their alkyl moieties. The expressions lower alkyl and lower alkoxy are intended to embrace groups containing from 1 to 6 carbon atoms and preferably 1 to 4 carbon atoms. The term halo means chloro, bromo, iodo or fluoro, preferably chloro or bromo.

The anti-secretory agents of this invention are active in the following scientifically recognized, standard test for gastic anti-secretory activity:

Male Charles River rats of Sprague-Dawley strain and 190 to 240 grams body weight are food deprived for 24 hours with water *ad libitum* until the test. Groups of ten rats each are assigned to either control or drug treatment. Pyloric ligation was performed under ether anaesthesia through a midline laparatomy, and either control vehicle (0.25 methylcellulose) or drug in control vehicle was administered intraduodenally. The rats are sacrificed by $CO_2$ asphyxiation four hours after pyloric ligation. The stomachs are removed and the gastric contents emptied into graduated centrifuge tubes. The gastric samples are centrifuged for 20 minutes and those obviously contaminated by food, blood or faeces are discarded. The volume of gastric fluid is recorded and the acid concentration of 1.0 milliliter sample aliquots is measured by electrometric titration to pH 7.0 with 0.1 N NaOH. The calculated product of gastric volume (mEq/L) estimates the total acid output (TAO, mEq/4 hr.) over the four-hour test period. An analysis of variance is performed on these data to determine statistically significant ($p<0.05$) deviation between control versus drug-treated groups.

In addition to anti-secretory activity, certain compounds of this invention possess diuretic activity and they are useful for treatment of conditions requiring diuresis therapy. The diuretic agents disclosed herein are those compounds in which the substituent in 4 position is primary amino. Thus, the compounds possessing diuretic activity may be depicted by the structural formula:

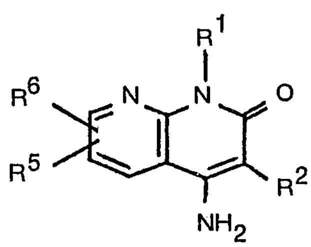

(III)

in which each of the R groups is defined in conjunction with the structural formula generically depicting compounds with anti-secretory activity. The 4-amino-1,8-naphthyridine derivatives demonstrated effectiveness generally comparable to hydrochlorothiazide in the standard, scientifically recognized test procedure of Lipschitz et al., J. Pharmacol. Exp. Therap., *79*, 97 (1945) wherein male Sprague-Dawley rats 14 to 17 weeks old (175-200 grams), after fasting for one day are given an oral physiological saline prime dose of 25 ml/kg. of the compound being tested. Each compound is given to 8 rats, urea at a dose of 960 mg/kg is given as a standard of comparison to 8 rats and saline alone is given to 8 more rats as a control. The animals are placed in metabolism cages, 2 rats per cage, and urine is collected for 3 hours. Volume of urine, sodium and potassium are determined and compared to the control to obtain the ratio of activity.

The dosage regimen for therapeutic use of the anti-secretory agents disclosed herein will vary with the mode of administration, size and age of the person under treatment as well as the severity of the dysfunction. Therefore, treatment of peptic ulcer disease must be individualized for the patient under the guidance of the attending physician. The use of the 4-amino-1,8-naphthyridines as diuretics must similarly be controlled in the human. Where two conditions occur simultaneously in the same patient, requiring treatment of peptic ulcer disease as well as diuresis, the 4-amino-1,8-naphthyridines afford the decided advantage of single compound treatment for both problems. Where the conditions occur separately, the second activity of the 4-amino-1,8-naphthyridines is not deleterious and not contraindicative of applicability of the treatment.

The compounds of this invention may be administered by conventional oral or parenteral routes as solids, liquids or isotonic solutions. Accordingly this invention also provides a pharmaceutical composition comprising a compound of formula I or a pharmaceutically acceptable salt thereof and a pharmaceutically acceptable carrier. Conventional adjuvants known to the art may be combined with

the compounds disclosed herein to provide compositions and solutions for administration purposes although it is considered desirable and feasible to use the compounds neat or pure without additives other than for the purpose of providing suitable pharmaceutically acceptable solid or liquid dosage units.

Intermediate 4-halo-naphthyridine precursors for production of the anti-secretory agents of this invention are compounds of the formula:

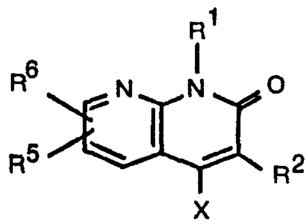

(IV)

in which $R^1$ is hydrogen, alkyl of 1 to 6 carbon atoms, cycloalkyl of 5 to 6 carbon atoms, alken-(3,4,5 or 6)-yl of 3 to 6 carbon atoms, or alkyn-(3,4,5 or 6)-yl of 3 to 6 carbon atoms;

$R^2$ is $-CCl_3$, $-CN$, $-CO_2H$, $CONR^8R^9$ or $-CO_2R^7$ whre $R^7$, $R^8$ and $R^9$ are as defined above;

X is chloro, bromo or iodo; and $R^5$ and $R^6$ are, independently hydrogen, halo, alkoxy of 1 to 6 carbon atoms, alkylamino of 1 to 4 carbon atoms, or alkyl of 1 to 4 carbon atoms. Compounds of formula IV and processes for preparing them are claimed in our copending European Patent Application No. 82200534.4 Publication No. 60012. The preferred intermediates are those conforming in structure to the preferred group of anti-secretory agents.

This invention also provides processes for preparing the compounds of the invention. Compounds of formula I may be prepared by a process which comprises

(a) reacting a 2-aminonicotinonitrile of formula

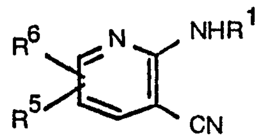

(V)

wherein $R^1$, $R^5$ and $R^6$ are as defined hereinbefore with (i) a lower alkyl malonyl halide followed by ring closure under basic conditions or (ii) an alkali metal di(lower)alkyl malonate ester, to given on acidification a compound of formula I wherein $R^2$ is $-CO_2R^7$ and $R^3$ and $R^4$ are both hydrogen, or

(b) reacting compound of formula

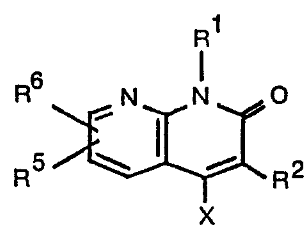

wherein $R^1$, $R^5$ and $R^6$ are as defined hereinabove and $R^2$ is $-CN$, $COOR^7$ or $-CONR^8R^9$ wherein $R^7$, $R^8$ and $R^9$ are as defined hereinabove and X is chlorine, bromine or iodine with an amine of formula $HNR^3R^4$ wherein $R^3$ and $R^4$ are as defined hereinabove to give a corresponding compound of formula I, or

(c) reacting a compound of formula

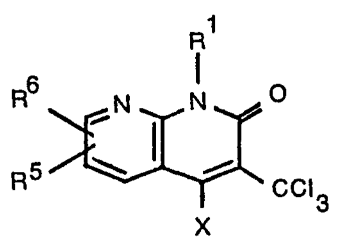

wherein X, $R^1$, $R^5$ and $R^6$ are as hereinbefore defined, with ammonia to give a compound of formula I wherein $R^3$ and $R^4$ are both hydrogen and $R^2$ is CN or $-CONH_2$, or

(d) alkylating, alkenylating or alkynylating an alkali metal salt of a compound of formula I wherein $R^1$ is an alkali metal to give a compound of formula I wherein $R^1$ is an alkali metal to give a compound of formula I wherein $R^1$ is alkyl, alkenyl or alkynyl as defined hereinabove, or

(e) converting an ester of formula I wherein $R^2$ is $-COOR^7$ to give an acid of formula I wherein $R^2$

is COOH or a pharmaceutically acceptable salt thereof, (e.g. alkali metal salt, ammonium or substituted ammonium salt), or

(f) converting a compound of formula I wherein $R^5$ and/or $R^6$ is halogen to a compound of formula I wherein $R^5$ and/or $R^6$ is lower alkoxy of 1 to 6 carbon atoms or lower alkylamino of 1 to 4 carbon atoms with simultaneous conversion of $R^2$ when $COOR^7$ to the corresponding lower alkylamide, or

(g) reacting a compound of formula I in which $R^2$ is $CO_2R^7$ with hydrazine, ammonia or an amine of formula $HNR^8R^9$ wherein $R^8$ and $R^9$ are as defined hereinabove to give a compound of formula I wherein $R^2$ is $—CONHNH_2$, $—CONH_2$ or $—CONR^8R^9$, or

(h) acylating a compound of formula I wherein $R^3$ and $R^4$ are both hydrogen to give a compound of formula I wherein $R^3$ and/or $R^4$ are alkanoyl of 2 to 4 carbon atoms or haloalkanoyl of 2 to 4 carbon atoms, or

(i) converting a compound of formula I to a pharmaceutically acceptable salt.

In process (b) the 4-substituted amino-3-carboxy or cyano-1,2-dihydro-2-oxo-1,8-naphthyridine derivatives of this invention are prepared by reacting a primary or secondary amine with an appropriately substituted 4-halo-naphthyridine precursor. By 4-halo is meant the chloro, bromo and iodo derivatives. The 4-halo-naphthyridine precursors are prepared by conventional techniques involving displacement of a 4-hydroxy group from the corresponding 4-hydroxy-naphthyridine with such halogenating reagents as phosphorus oxychloride or thionyl chloride to obtain the 4-chloro-naphthyridines and phosphorus oxybromide to obtain the 4-bromo-naphthyridines. The 4-iodo-naphthyridines are prepared by reaction of a 4-chloro-naphthyridine with sodium iodide in an appropriate inert solvent such as acetone.

Alternatively, the 4-chloro, bromo or iodo-naphthyridines may also be prepared directly by nitrosation of the appropriately ring substituted 4-amino-naphthyridines in hydrochloric, hydrobromic or hydroiodic acid, respectively.

With reference to process (a) above, the 4-amino-3-carboxy-1,2-dihydro-2-oxo-1,8-naphthyridine derivatives of this invention can be prepared directly from a 2-substituted amino nicotinonitrile reacting with a lower alkyl malonyl chloride followed by a Dieckman ring closure with base, e.g. sodium alkoxide, or an analogous strong base such as potassium alkoxide or NaH or $NaNH_2$ in an aprotic solvent, for example:

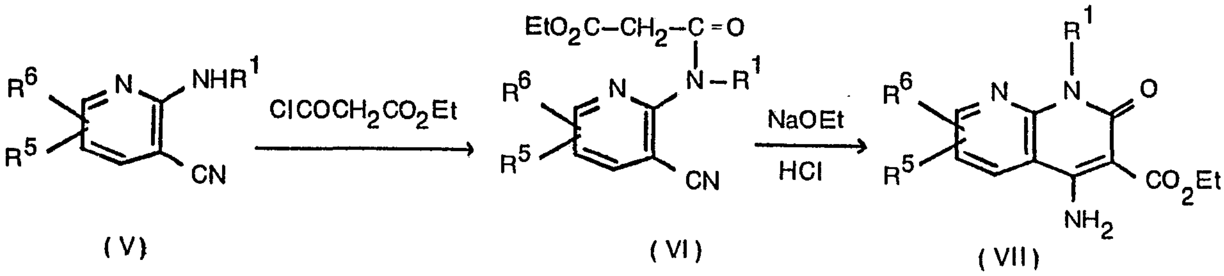


(V) (VI) (VII)

Alternatively, the 2-substituted aminonicotinonitrile may be reacted with the sodio salt of a di-lower alkyl malonate to give directly on acidification the 4-amino-3-carboxy-1,2-dihydro-2-oxo-1,8-naphthyridine derivative, for example

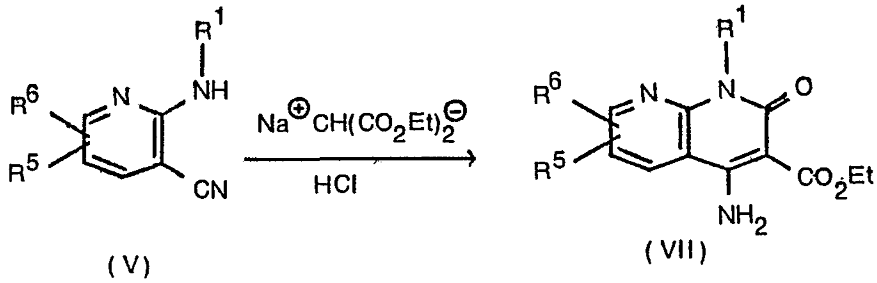


(V) (VII)

With reference to process (d) above alkylation, alkenylation or alkynylation may be effected by, for example, treating a compound of formula I wherein $R^1$ is hydrogen, with an alkali metal alkoxide, e.g. NaOEt to give an alkali metal salt ($R^1$=alkali metal and reacting this with a lower alkyl, alkenyl or alkynyl iodide, bromide or chloride (RX where R contains 1 to 6 carbon atoms) to afford alternatively the 1-alkyl-, alkenyl- or alkynyl-4-amino-3-carboxy-1,2-dihydro-2-oxo-1,8-naphthyridine derivatives described above. This process is illustrated by the following reaction:

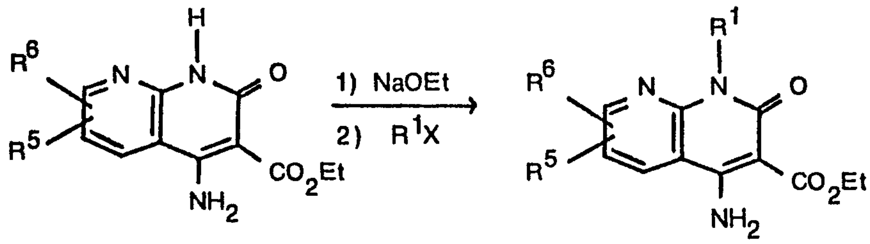


(VIII) (IX)

In process (g) above the amide substituent

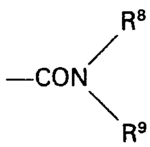

representing $R^2$ is readily produced at 3-position of the 4-amino and 4-substituted amino-3-alkoxycarbonyl-1,8-naphthyridine derivatives by reaction of the ester $—CO_2R^7$ with ammonia or a primary or secondary alkylamine by conventional techniques in which the alkyl group(s) independently contain from 1 to 6 carbon atoms. Similarly the hydrazide ($R^2—CONHNH_2$) is prepared by treatment with hydrazine.

In process (c) above the 4-amino-3-carbamoyl or cyano-1,2-dihydro-2-oxo-1,8-naphthyridine derivatives may also be prepared by reaction of ammonia with an appropriately substituted 3-trichloromethyl-4-halo-1,8-naphthyridine derivative, and is illustrated by the following reaction:

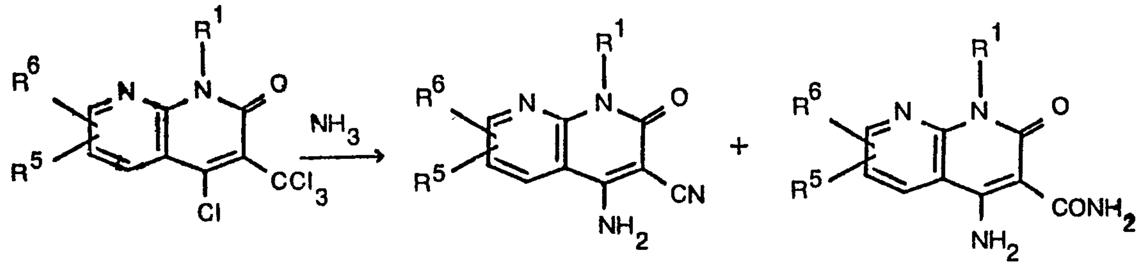


(X) (XI) (XII)

The 3-trichloromethyl-4-chloro-1,8-naphthyridine precursor is obtained by reaction of the corresponding 3-carboethoxy-4-hydroxy-naphthyridine with $PCl_5$, as shown below:

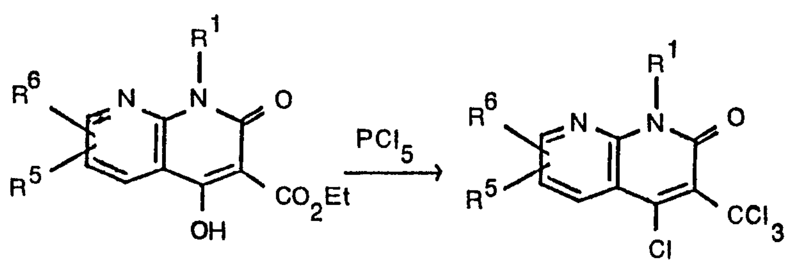


(XIII) (X)

In process (f) halo substituents for $R^5$, $R^6$ may be replaced by lower alkoxy, e.g. by reaction with an alkali metal alkoxide, e.g. NaOEt. They may also be replaced by lower alkylamino by reaction with a lower alkylamine, e.g. ethylamine, which simultaneously converts an ester $R^2$ group when present to the corresponding lower alkylamide.

In process (h) the 4-amino substituent may be mono acylated or diacylated using an acylating agent such as an acid halide (e.g. acetyl chloride, chloracetyl chloride) or an anhydride.

The following examples illustrate the preparation of the compounds of the invention. An index of drug gastric anti-secretory activity is reported at the end of each example illustrating its production. These data, expressed as percentage inhibition, show the reduction of acid secretion in drug-treated compared to control groups based upon i.d. administration of 32 mg/kg. of the tested compound.

Example 1

4-Amino-1,2-Dihydro-2-Oxo-1,8-Naphthyridine-3-Carboxylic Acid Ethyl Ester

To a solution of 4.14 g. (0.18 g. atom) of sodium in 100 ml. of ethanol was added 28.8 g. (0.18 mole) of diethyl malonate. After stirring at room temperature for 5 minutes, 7.14 g. (0.06 mole) of 2-aminonicotinonitrile was added and the mixture was heated under reflux for 6 hours. The mixture was cooled and was diluted with 100 ml. of water and was acidified with conc. hydrochloric acid. On cooling, a precipitate was formed which was collected and was triturated with 1000 ml. of boiling ethanol. The mixture was filtered and the filtrate was cooled in ice to precipitate 2.9 g. of product, m.p. 264—267°C.

| | | | |
|---|---|---|---|
| Analysis for: | $C_{11}H_{11}N_3O_3$ | | |
| Calculated: | C, 56.65; | H, 4.75; | N, 18.02 |
| Found: | C, 56.37; | H, 4.79; | N 18.08 |

Percentage Inhibition: 68%

Example 2

4-Amino-1,2-Dihydro-2-Oxo-1,8-Naphthyridine-3-Carboxylic Acid

The material which was insoluble in boiling ethanol from the previous reaction was recrystallized from, N,N-dimethylformamide to give a small amount of the corresponding carboxylic acid, m.p. 296—298°C. dec.

| | | | |
|---|---|---|---|
| Analysis for: | $C_9H_7N_3O_3$ | | |
| Calculated: | C, 52.68; | H, 3.44; | N, 20.48 |
| Found: | C, 52.27; | H, 3.72; | N, 20.23 |

Example 3

1-Methyl-1,2-Dihydro-2-Oxo-4-(1-Pyrrolidinyl)-1,8-Naphthyridine-3-Carboxylic Acid Ethyl Ester

A stirred mixture of 2.7 g. (0.01 mole) of 4-chloro-1,2-dihydro-1-methyl-2-oxo-1,8-naphthyridine-3-carboxylic acid ethyl ester, 0.7 g. (0.01 mole) of pyrrolidine and 1.06 g. (0.01 mole) of sodium carbonate in 25 ml. of ethanol was heated under reflux for 5 hours. The mixture was filtered and the filtrate was cooled in ice. The precipitate which formed was collected and was dissolved in 20 ml. of ethyl acetate. The solution was diluted with petroleum ether to the cloudy point. The precipitate which formed was collected to yield 0.5 g. of product, m.p. 118—120°C.

| | | | |
|---|---|---|---|
| Analysis for: | $C_{16}H_{19}N_3O_3$ | | |
| Calculated: | C, 63.77; | H, 6.36; | N, 13.95 |
| Found: | C, 63.46; | H, 6.51; | N. 14.01 |

Percentage Inhibition: 55%.

Example 4

1,7-Dimethyl-1,2-Dihydro-2-Oxo-4-(1-Pyrrolidinyl)-1,8-Naphthyridine-3-Carboxylic Acid Ethyl Ester

4-Chloro-1,7-dimethyl-1,2-dihydro-2-oxo-1,8-naphthyridine-3-carboxylic acid ethyl ester produced by the reaction of thionyl chloride and the corresponding 4-hydroxy precursor in a manner analogous to the procedure of Example 1 of E.P. Patent Application No. 82200534,4 (Publication No. 60012) was reacted with pyrrolidine as in Example 3 to yield the title compound, m.p. 108—111°C.

| | | | |
|---|---|---|---|
| Analysis for: | $C_{17}H_{21}N_3O_3$ | | |
| Calculated: | C, 64.74; | H, 6.71; | N, 13.33 |
| Found: | C, 64.78; | H, 6.66; | N, 13.40 |

Percentage Inhibition: 78%

Example 5

1-Methyl-1,2-Dihydro-4-(4-Methyl-1-Piperazinyl)-2-Oxo-1,8-Naphthyridine-3-Carboxylic Acid Ethyl Ester

The title compound was prepared by the procedure of Example 3, employing N-methylpiperazine in lieu of pyrrolidine as the amine reactant, m.p. 231—234°C. dec.

| | | | |
|---|---|---|---|
| Analysis for: | $C_{17}H_{23}ClN_4O_3 \cdot \frac{1}{2} H_2O$ | | |
| Calculated: | C, 54.33; | H, 6.44; | N, 14.91 |
| Found: | C, 54.19; | H, 6.31; | N, 14.74 |

Percentage Inhibition: 74%

Example 6

1,7-Dimethyl-1,2-Dihydro-4-(4-Methyl-1-Piperazinyl)-2-Oxo-1,8-Naphthyridine-3-Carboxylic Acid Ethyl Ester

The title compound was prepared by the procedure of Example 4, substituting N-methylpiperazine for pyrrolidine, m.p. 278—280°C. dec.

| | | | |
|---|---|---|---|
| Analysis for: | $C_{18}H_2ClN_4O_3.HCl.\frac{1}{2}H_2O$ | | |
| Calculated: | C, 55.45; | H, 6.72; | N, 14.37 |
| Found: | C, 55.66; | H, 6.51; | N, 14.50 |

Percentage Inhibition: 98%

Example 7

4-Amino-1,2-Dihydro-1-Methyl-2-Oxo-1,8-Naphthyridine-3-Carboxylic Acid Ethyl Ester

The title compound was prepared by reacting ethyl malonyl chloride with 2-methylamino-nicotinonitrile following the procedure of Example 8A below, m.p. 203—206°C.

| | | | |
|---|---|---|---|
| Analysis for: | $C_{12}H_{13}N_3O_3$ | | |
| Calculated: | C, 58.29; | H, 5.30; | N, 17.00 |
| Found: | C, 57.96; | H, 5.31; | N, 17.16 |

Percentage Inhibition: 86%

Example 8

4-Amino-1-Ethyl-1,2-Dihydro-2-Oxo-1,8-Naphthyridine-3-Carboxylic Acid Ethyl Ester

A) Following the procedure of Taylor et al., J. Org. Chem., *19*, 1633 (1954), a stirred mixture of 14 g. of nicotinamide N-oxide, 29.7 g. of phosphorus pentachloride and 40 ml. of phosphorus oxychloride was heated under reflux for 2 hours. The phosphorus oxychloride was evaporated in a rotary evaporator and the residue was poured over ice. The insoluble material was collected, air dried and was recrystallized from heptane to give 6.0 g. of 2-chloronicotinonitrile.

A stirred mixture of 4 g. of 2-chloronicotinonitrile in 200 ml. of a saturated ethanolic ethylamine solution was heated under reflux for 5 hours. The solution was cooled and was diluted with 400 ml. of water. The precipitate of 2-ethylaminonicotinonitrile which formed was collected, air dried and was used directly in the next step without further purification.

To a solution of 4.4 g. (0.03 mole) of 2-ethylaminonicotinonitrile in 200 ml. of anhydrous diethyl ether was added 2.25 g. (0.015 mole) of ethyl malonyl chloride. After stirring at room temperature for 2 hours, the mixture was filtered. The filtrate was evaporated in a rotary evaporator and the residue was dissolved in 20 ml. of ethanol. This solution was added to a solution of 0.69 g. (0.03 g. atom) of sodium in 100 ml. of ethanol. After stirring for 5 minutes at room temperature, the mixture was diluted with water and was acidified with conc. hydrochloric acid. The precipitate which formed was collected, air dried and was recrystallized from ethyl acetate to afford 1.8 g. of the title compound as a hemihydrate, m.p. 205—208°C.

B) Alternatively, 4-amino-1-ethyl-1,2-dihydro-2-oxo-1,8-naphthyridine-3-carboxylic acid ethyl ester may be prepared as follows:

To a solution of 2.07 g. (0.09 g. atom) of sodium in 75 ml. of absolute ethanol was added 14.4 g. (0.09 mole) of diethyl malonate. The solution was stirred at room temperature for 5 minutes. Then 4.4 g. (0.03 mole) of 2-ethylaminonicotinonitrile was added and the mixture was heated under reflux for 6 hours. The mixture was cooled and was diluted with 75 ml. of water and was acidified with concentrated hydrochloric acid. The precipitate which formed was collected and was dried to give 3.2 g. of material. Two recrystallizations from ethanol provided 1.1 g. of pure product, m.p. 203—207°C.

C) A third method of preparing 4-amino-1-ethyl-1,2-dihydro-2-oxo-1,8-naphthyridine-3-carboxylic acid ethyl ester is as follows:

To a stirred solution of 0.026 mole of sodium ethoxide (0.6 g. sodium in 200 ml. of ethanol) was added 6 g. (0.025 mole) of 4-amino-1,2-dihydro-2-oxo-1,8-naphthyridine-3-carboyxlic acid ethyl ester and 11.7 g. (0.075 mole) of ethyl iodide. The reaction mixture was heated under reflux for 4 hours; then filtered. The filtrate was taken to dryness and the residue was washed with water. The crude product amounted to 2.3 g., m.p. 194—198° and gave no melting point depression on admixture with samples prepared as previously described.

| | | | |
|---|---|---|---|
| Analysis for: | $C_{13}H_{15}N_3O_3.\frac{1}{2}H_2O$ | | |
| Calculated: | C, 57.77; | H, 5.97; | N, 15.55 |
| Found: | C, 57.82; | H, 5.97; | N, 15.81 |

Percentage Inhibition: 89%

Example 9

4-Amino-1-Ethyl-1,2-Dihydro-2-Oxo-1,8-Naphthyridine-3-Carboxylic Acid, n-Butyl Ester

The title compound was prepared from sodio di-n-butylmalonate and 2-ethylaminonicotinonitrile following the procedure of Example 8B, m.p. 117—120°C

| | | | |
|---|---|---|---|
| Analysis for: | $C_{15}H_{19}N_3O_3$ | | |
| Calculated: | C, 62.26; | H, 6.62; | N, 14.52 |
| Found: | C, 61.96; | H, 6.50; | N, 14.74 |

Percentage Inhibition 85%

Example 10

4-Amino-1,2-Dihydro-1-Ethyl-2-Oxo-1,8-Naphthyridine-3-Carboxylic Acid Hydrazide

A stirred mixture of 2 g. of 4-amino-1-ethyl-1,2-dihydro-2-oxo-1,8-naphthyridine-3-carboxylic acid ethyl ester in 20 ml. of ethanol containing 5 ml. of hydrazine was heated under reflux for 4 hours. The mixture was cooled and the insoluble material was collected. The filter cake was recrystallized from ethanol to produce 1.1 g. of product, m.p. 275—277°C. dec.

| | | | |
|---|---|---|---|
| Analysis for: | $C_{11}H_{13}N_5O_2$ | | |
| Calculated: | C, 53.43; | H, 5.30; | N, 28.33 |
| Found: | C, 53.33; | H, 5.47; | N, 28.69 |

Percentage Inhibition: 88%

Example 11

4-Amino-1-Ethyl-1,2-Dihydro-2-Oxo-1,9-Naphthyridine-3-Carboxylic Acid

A stirred mixture of 2 g. of 4-amino-1-ethyl-1,2-dihydro-2-oxo-1,8-naphthyridine-3-carboxylic acid ethyl ester in 20 ml. of 10% aqueous sodium hydroxide containing 5 ml. of ethanol was heated under reflux for 3 hours to saponify the ester. The solution was cooled and was acidified with glacial acetic acid. The precipitate which formed was collected, air dried, and was recrystallized from ethanol to give 0.4 g. of product, m.p. 245—248°C.

| | | | |
|---|---|---|---|
| Analysis for: | $C_{11}H_{11}N_3O_3$ | | |
| Calculated: | C, 56.65; | H, 4.75; | N, 18.02 |
| Found: | C, 56.41; | H, 4.80; | N, 18.04 |

Percentage Inhibition: 76%

Treatment of the free carboxylic acid with a stoichiometric amount of NaOH, KOH, $NH_4OH$, diethylamine, and the like bases forms the corresponding carboxylic acid salt.

Example 12

4-(Diacetylamino)-1-Ethyl-1,2-Dihydro-2-Oxo-1,8-Naphthyridine-3-Carboxylic Acid Ethyl Ester

A stirred mixture of 6 g. of 4-amino-1-ethyl-1,2-dihydro-2-oxo-1,8-naphthyridine-3-carboxylic acid ethyl ester in 100 ml. of acetyl chloride was heated under reflux for 24 hours. The mixture was filtered and the filtrate was evaporated in a rotary evaporator. The residue was recrystallized from 20 ml. of ethyl acetate to afford 2.9 g. of product, m.p. 133—135°C.

| | | | |
|---|---|---|---|
| Analysis for: | $C_{17}H_{19}N_3O_5$ | | |
| Calculated: | C, 59.12; | H, 5.55; | N, 12.17 |
| Found: | C, 59.05; | H, 5.55; | N, 12.56 |

Percentage Inhibition: 47%

Example 13

1-Ethyl-1,2-Dihydro-4-(4-Methyl-1-Piperazinyl)-2-Oxo-1,8-Naphthyridine-3-Carboxylic Acid Ethyl Ester

The title compound was prepared in accordance with the procedure of Example 3, employing 4-chloro-1-ethyl-1,2-dihydro-2-oxo-1,8-naphthyridine-3-carboxylic acid ethyl ester and N-methylpiperazine as the reactants, m.p. 230—233°C.

| | | | |
|---|---|---|---|
| Analysis for: | $C_{18}H_{25}ClN_4O_3$ | | |
| Calculated: | C, 56.76; | H, 6.62; | N, 14.71 |
| Found: | C, 56.49; | H, 6.59; | N, 14.50 |

Percentage Inhibition: 94%

Example 14

4-Amino-1-Propyl-1,2-Dihydro-2-Oxo-1,8-Naphthyridine-3-Carboxylic Acid Ethyl Ester

A title compound was prepared following the procedure of Example 8A, employing 2-propylaminonicotinonitrile and ethyl malonyl chloride with reactants, m.p. 165—168°C.

| | | | |
|---|---|---|---|
| Analysis for: | $C_{14}H_{17}N_3O_3$ | | |
| Calculated: | C, 61.08; | H, 6.22; | N, 15.26 |
| Found: | C, 61.02; | H, 6.04; | N, 15.19 |

Percentage Inhibition: 79%

Example 15

4-Amino-1-Isobutyl-1,2-Dihydro-2-Oxo-1,8-Naphthyridine-3-Carboxylic Acid Ethyl Ester

The title compound was prepared following the procedure of Example 8A, employing 2-isobutylaminonicotinonitrile and ethyl malonyl chloride as the reactants, m.p. 157—159°C.

| | | | |
|---|---|---|---|
| Analysis for: | $C_{15}H_{19}N_3O_3$ | | |
| Calculated: | C, 62.26; | H, 6.62; | N, 14.52 |
| Found: | C, 62.03; | H, 6.55; | N, 14.56 |

Percentage Inhibition: 81%

Example 16

4-Amino-1-Cyclohexyl-1,2-Dihydro-2-Oxo-1,8-Naphthyridine-3-Carboxylic Acid Ethyl Ester

The title compound was prepared following the procedure of Example 8A, employing 2-cyclohexylaminonicotinonitrile and ethyl malonyl chloride as the reactants, m.p. 172—175°C.

| | | | |
|---|---|---|---|
| Analysis for: | $C_{17}H_{21}N_3O_3$ | | |
| Calculated: | C, 64.74; | H, 6.71; | N, 13.33 |
| Found: | C, 64.36; | H, 6.67; | N, 12.99 |

Percentage Inhibition: 68%

Example 17

1-Allyl-4-Amino-1,2-Dihydro-2-Oxo-1,8-Naphthyridine-3-Carboxylic Acid Ethyl Ester

The title compound was prepared following the procedure of Example 8A, employing 2-allylaminonicotinonitrile and ethyl malonyl chloride as the reactants, m.p. 161—164°C.

| | | | |
|---|---|---|---|
| Analysis for: | $C_{14}H_{15}N_3O_3$ | | |
| Calculated: | C, 61.53; | H, 5.53; | N, 15.38 |
| Found: | C, 61.29; | H, 5.52; | N, 15.48 |

Percentage Inhibition: 85%

Example 18

1-Allyl-1,2-Dihydro-7-Methyl-2-Oxo-4-(Pyrrolidinyl)-1,8-Naphthyridine-3-Carboxylic Acid Ethyl Ester

The title compound was prepared by reaction of 1-allyl-4-chloro-1,2-dihydro-7-methyl-2-oxo-1,8-naphthyridine-3-carboxylic acid ethyl ester and pyrrolidine following the procedure of Example m.p. 97—103°C.

| | | | |
|---|---|---|---|
| Analysis for: | $C_{19}H_{23}N_3O_3$ | | |
| Calculated: | C, 66.84; | H, 6.79; | N, 12.31 |
| Found: | C, 66.73; | H, 6.88; | N, 12.28 |

Percentage Inhibition: 46%

Example 19

4-[(Chloroacetyl)Amino]-1,2-Dihydro-2-Oxo-1-(2-Propenyl)-1,8-Naphthyridine-3-Carboxylic Acid

The title compound was prepared by reaction of chloroacetyl chloride with the product of Example 17 under reflux for one hour, m.p. 145—148°C.

| | | | |
|---|---|---|---|
| Analysis for: | $C_{14}H_{12}ClN_3O_4$ | | |
| Calculated: | C, 52.33; | H, 3.77; | N, 13.08 |
| Found: | C, 51.99; | H, 3.75; | N, 13.15 |

Percentage Inhibition: 41%

Example 20

1-Ethyl-1,2-Dihydro-4-Trifluoroacetylamino-2-Oxo-1,8-Naphthyridine-3-Carboxylic Acid Ethyl Ester

The title compound was produced by reaction of trifluoroacetic acid anhydride with the product of Example 8 under reflux for three hours, m.p. 195—197°C.

| | | | |
|---|---|---|---|
| Analysis for: | $C_{15}H_{14}F_3N_3O_4$ | | |
| Calculated: | C, 50.42; | H, 3.94; | N, 11.76 |
| Found: | C, 50.25; | H, 3.91; | N, 11.77 |

Percentage Inhibition: 88%

Example 21

1-Propargyl-4-Amino-2-Oxo-1,8-Naphthyridine-3-Carboxylic Acid Ethyl Ester

The title compound is produced following the procedure of Example 8A with the exception that propargylamine is employed as an initial reactant rather than ethylamine.

Example 22

4-Amino-7-Chloro-1-Ethyl-1,2-Dihydro-2-Oxo-1,8-Naphthyridine-3-Carboxylic Acid Ethyl Ester

The title compound was prepared by reaction of 6-chloro-2-ethylaminonicotinonitrile and sodio diethylmalonate following the procedure of Example 1.

Example 23

4-Amino-7-Ethoxy-1-Ethyl-1,2-Dihydro-2-Oxo-1,8-Naphthyridine-3-Carboxylic Acid Ethyl Ester

The title compound was prepared by reaction of sodium ethoxide with the product of Example 22.

Example 24

4-Amino-1,N-Diethyl-7-Ethylamino-1,2-Dihydro-2-Oxo-1,8-Naphthyridine-3-Carboxamide

The title compound is prepared by reaction of ethylamine with the product of Example 22.

Example 25

4-Amino-1-Ethyl-1,2-Dihydro-7-Methyl-2-Oxo-1,8-Naphthyridine-3-Carboxylic Acid Ethyl Ester

The title compound was prepared by reaction of 2-ethylamino-6-methylnicotinonitrile and ethyl malonyl chloride following the procedure of Example 8A, m.p. 196°C.

| | | | |
|---|---|---|---|
| Analysis for: | $C_{14}H_{17}N_3O_3$ | | |
| Calculated: | C, 61.08; | H, 6.22; | N, 15.26 |
| Found: | C, 60.96; | H, 6.18; | N, 15.35 |

Percentage Inhibition: 76%

Example 26

4-Amino-1-Ethyl-1,2-Dihydro-7-Methyl-2-Oxo-1,8-Naphthyridine-3-Carbonitrile

A stirred mixture of 7.5 g. of 4-chloro-1-ethyl-7-methyl-3-(trichloromethyl)-1,8-naphthyridin-2-(1H)-one was heated under reflux in 200 ml. of a saturated ethanolic ammonia solution for 6 hours. The mixture was cooled and the precipitate which formed was collected and was recrystallised twice from ethanol to give 0.3 g. of the title compound, m.p. >300°C.

| | | | |
|---|---|---|---|
| Analysis for: | $C_{12}H_{12}N_4O$ | | |
| Calculated: | C, 63.14; | H, 5.30; | N, 24.55 |
| Found: | C, 62.96; | H, 5.47; | N, 24.64 |

Percentage Inhibition: 53%

Example 27

4-Amino-1-Ethyl-1,2-Dihydro-7-Methyl-2-Oxo-1,8-Naphthyridine-3-Carboxamide

The reaction filtrate from the previous example was diluted with water. The precipitate which formed was collected, air dried and was recrystallized from ethanol to give 0.2 g. of product, m.p. 263—266°C.

| | | | |
|---|---|---|---|
| Analysis for: | $C_{12}H_{14}N_4O_2$ | | |
| Calculated: | C, 58.52; | H, 5.73; | N, 22.75 |
| Found: | C, 58.38; | H, 5.39; | N, 22.37 |

Percentage Inhibition: 57%

Example 28

1-Ethyl-1,2-Dihydro-7-Methyl-2-Oxo-4-(1-Pyrrolidinyl)-1,8-Naphthyridine-3-Carbonitrile

The title compound was prepared by reaction of 4-chloro-1-ethyl-1,2-dihydro-7-methyl-2-oxo-1,8-naphthyridine-3,-carbonitrile, (prepared by reacting 1-ethyl-1,2-dihydro-4-hydroxy-7-methyl-2-oxo-1,8-naphthyridine-3-carboxylic acid ethyl ester with ethanolic ammonia to form the 3-carboxamide, and that product with phosphorus oxychloride), with pyrrolidine following the procedure of Example 3, m.p. 211—213°C.

| | | | |
|---|---|---|---|
| Analysis for: | $C_{16}H_{18}N_4O$ | | |
| Calculated: | C, 68.06; | H, 6.43; | N, 19.85 |
| Found: | C, 68.24; | H, 6.57; | N, 19.84 |

Percentage Inhibition: 41%

Example 29

1-Ethyl-1,2-Dihydro-7-Methyl-2-Oxo-4-(1-Pyrrolidinyl)-1,8-Naphthyridine-3-Carboxylic Acid Ethyl Ester

The title compound was prepared following the procedure of Example 3, from pyrrolidine and 4-chloro-1-ethyl-1,2-dihydro-7-methyl-2-oxo-1,8-naphthyridine-3-carboxylic acid ethyl ester, the latter compound being prepared from the corresponding 4-hydroxy compound and thionyl chloride as in Example 4 or by nitrosation of the corresponding 4-amino compound with $NaNO_2$ and NCl, m.p. 113—115°C.

| | | | |
|---|---|---|---|
| Analysis for: | $C_{18}H_{23}N_3O_3$ | | |
| Calculated: | C, 65.63; | H, 7.04; | N, 12.76 |
| Found: | C, 65.67; | H, 6.97; | N, 12.82. |

Percentage Inhibition: 83%

Example 30

4-Diethylamino-1-Ethyl-1,2-Dihydro-7-Methyl-2-oxo-1,8-Naphthyridine-3-Carboxylic Acid Ethyl Ester

Following the procedure of Example 29, employing diethylamine in lieu of pyrrolidine, yields the title compound, m.p. 79—82°C.

| | | | |
|---|---|---|---|
| Analysis for: | $C_{18}H_{25}N_3O_3$ | | |
| Calculated: | C, 65.23; | H, 7.60; | N, 12.68 |
| Found: | C, 64.89; | H, 7.40; | N, 12.58. |

Percentage Inhibition: 50%

Example 31

1-Ethyl-1,2-Dihydro-7-Methyl-4-(4-Morpholinyl)-2-Oxo-1,8-Naphthyridine-3-Carboxylic Acid Ethyl Ester

Morpholine was substituted for pyrrolidine in the procedure of Example 29 to yield the title compound, m.p. 129—131°C.

| | | | |
|---|---|---|---|
| Analysis for: | $C_{18}H_{23}N_3O_4$ | | |
| Calculated: | C, 62.52; | H, 6.71; | N, 12.17 |
| Found: | C, 62.47; | H, 6.46; | N, 11.99 |

Percentage Inhibition: 88%

Example 32

1-Ethyl-1,2-Dihydro-7-Methyl-4-(4-Methyl-1-Piperazinyl)-2-Oxo-1,8-Naphthyridine-3-Carboxylic Acid Ethyl Ester Hydrochloride

N-Methylpiperazine was substituted for pyrrolidine in the procedure of Example 29 to yield the title compound, m.p. 285—288°C. dec.

| | | | |
|---|---|---|---|
| Analysis for: | $C_{19}H_{27}N_4O_3Cl$ | | |
| Calculated: | C, 57.59; | H, 6.89; | N, 14.19 |
| Found: | C, 57.75; | H, 7.04; | N, 14.27. |

Percentage Inhibition: 85%

The product of the preceding paragraph was converted to its free base with excess sodium carbonate and then saponified with a stoichiometric amount of NaOH to yield the sodium salt of the carboxylic acid. Similarly the ethyl ester may be saponified with a stoichiometric amount of potassium hydroxide. Other salts may be produced directly from the free carboxylic acid by conventional means.

Example 33

1-Ethyl-1,2-Dihydro-7-Methyl-2-Oxo-4-(4-Phenyl-1-Piperazinyl)-1,8-Naphthyridine-3-Carboxylic Acid Ethyl Ester

Following the procedure of Example 29, with the exception that 1-phenylpiperazine is substituted for pyrolidine, the title compound is produced, m.p. 110—112°C.

| | | | |
|---|---|---|---|
| Analysis for: | $C_{24}H_{28}N_4O_3$ | | |
| Calculated: | C, 68.55; | H, 6.71; | N, 13.33 |
| Found: | C, 68.34; | H, 6.64; | N, 13.35. |

Example 34

1-Ethyl-1,2-Dihydro-4-[(2-Hydroxyethyl)Methylamino]-7-Methyl-2-Oxo-1,8-Naphthyridine-3-Carboxylic Acid Ethyl Ester

The title compound was produced by the procedure of Example 29, substituting 2-methylaminoethanol for pyrolidine, m.p. 111—113°C.

| | | | |
|---|---|---|---|
| Analysis for: | $C_{17}H_{23}N_3O_4$ | | |
| Calculated: | C, 61.24; | H, 6.95; | N, 12.61 |
| Found; | C, 61.09; | H, 6.81; | N, 12.63. |

Percentage Inhibition: 52%

Example 35

1-Ethyl-1,2-Dihydro-4-(3-Dimethylaminopropylamino)-7-Methyl-2-Oxo-1,8-Naphthyridine-3-Carboxylic Acid Ethyl Ester

The title compound was produced by the procedure of Example 29, substituting 3-dimethylaminopropylamine for pyrrolidine, m.p. 75°C.

| | | | |
|---|---|---|---|
| Analysis for: | $C_{19}H_{31}ClN_4O_4$ | | |
| Calculated: | C, 55.00; | H, 7.53; | N, 13.50 |
| Found: | C, 55.25; | H, 7.37; | N, 13.59 |

Percentage Inhibition: 32%

Example 36

1-Ethyl-1,2-Dihydro-4-Ethylamino-7-Methyl-2-Oxo-1,8-Naphthyridine-3-Carboxylic Acid Ethyl Ester

The title compound was produced by the procedure of Example 30, substituting ethylamine for pyrrolidine m.p. 133—136°C.

| | | | |
|---|---|---|---|
| Analysis for: | $C_{16}H_{21}O_3N_3$ | | |
| Calculated: | C, 63.35; | H, 6.98; | N, 13.85 |
| Found: | C, 63.25; | H, 7.19; | N, 13.87. |

Percentage Inhibition: 70%

Example 37

1-Ethyl-1,2-Dihydro-7-Methyl-2-Oxo-4-(1-Piperazinyl)-1,8-Naphthyridine-3-Carboxylic Acid Ethyl Ester

The procedure of Example 29 was repeated, substituting piperazine for pyrrolidine, to yield the title compound, m.p. 137—139°C.

| | | | |
|---|---|---|---|
| Analysis for: | $C_{18}H_{24}N_4O_3$ | | |
| Calculated: | C, 62.77; | H, 7.02; | N, 16.27 |
| Found: | C, 62.76; | H, 6.83; | N, 16.12. |

Percentage Inhibition: 98%

Example 38

4,4-(1,4-Piperazinediyl)Bis[1-Ethyl-1,2-Dihydro-7-Methyl-2-Oxo-1,8-Naphthyridine-3-Carboxylic Acid]Diethyl Ester

The procedure of Example 29 was repeated, employing 1-ethyl-1,2-dihydro-7-methyl-2-oxo-(1-piperazinyl)-1,8-naphthyridine-3-carboxylic acid ethyl ester as the amine reactant in lieu of pyrrolidine, m.p. 264—267°C.

| | | | |
|---|---|---|---|
| Analysis for: | $C_{32}H_{38}N_6O_6$ | | |
| Calculated: | C, 63.77; | H, 6.36; | N, 13.95 |
| Found: | C, 63.38; | H, 6.22; | N, 13.75. |

Percentage Inhibition: 21%

Example 39

1-Ethyl-1,2-Dihydro-4-(4-Carbethoxy-1-Piperazinyl)-7-Methyl-2-Oxo-1,8-Naphthyridine-3-Carboxylic Acid Ethyl Ester

Following the procedure of Example 29, the title compound was prepared by substituting ethyl N-piperazino carboxylate as the amine reactant rather than pyrrolidine, to obtain the title compound, m.p. 138—140°C.

| | | | |
|---|---|---|---|
| Analysis for: | $C_{21}H_{28}N_4O_5$ | | |
| Calculated: | C, 60.56; | H, 6.78; | N, 13.45 |
| Found: | C, 60.47; | H, 7.04; | N, 13.53. |

**Claims for the Contracting States: BE CH DE FR IT LU NL SE LI**

1. A compound of the formula:

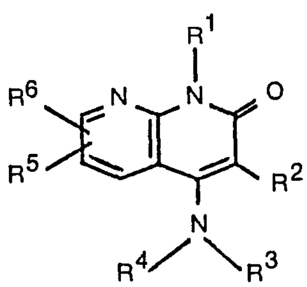

(I)

in which $R^1$ is hydrogen, alkyl of 1 to 6 carbon atoms, cycloalkyl of 5 to 6 carbon atoms, alken-(3,4,5 or 6)-yl of 3 to 6 carbon atoms or alkyn-(3,4,5 or 6)-yl of 3 to 6 carbon atoms; $R^2$ is —CN, —$CO_2H$, —$CO_2R^7$ where $R^7$ is alkyl of 1 to 6 carbon atoms, —$CONHNH_2$ or

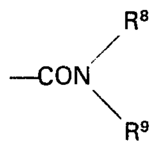

where $R^8$ and $R^9$ are independently hydrogen or alkyl of 1 to 6 carbon atoms; $R^3$ and $R^4$ are independently hydrogen, alkyl of 1 to 6 carbon atoms, hydroxyalkyl of 1 to 6 carbon atoms, dialkylaminoalkyl of 4 to 8 carbon atoms, alkanoyl of 2 to 4 carbon atoms, haloalkanoyl of 2 to 4 carbon atoms, or when taken together with the nitrogen atom to which they are attached, $R^3$ and $R^4$ complete a heterocyclic group selected from aziridinyl, azetidinyl, pyrrolidinyl, piperidinyl, piperazinyl, 4-lower alkylpiperazinyl, 4-phenylpiperazinyl, 4-[1-ethyl-1,2-dihydro-7-methyl-2-oxo-1,8-naphthyridine-4-yl-3-carboxylic acid ethyl ester]piperazinyl, 4-carb(lower)alkoxy-piperazinyl, morpholinyl, thiomorpholinyl or a ring substituted lower alkyl analogue thereof; $R^5$ and $R^6$ are independently hydrogen, alkyl of 1 to 4 carbon atoms, halo, alkylamino of 1 to 4 carbon atoms, or alkoxy of 1 to 6 carbon atoms or a pharmaceutically acceptable salt thereof; the term "lower" used in connection with alkyl or alkoxy meaning such groups contain 1 to 6 carbon atoms.

2. A compound of formula I as claimed in Claim 1 wherein $R^1$ and $R^7$ are alkyl of 1 to 4 carbon atoms.

3. A compound of formula I as claimed in Claim 1 or Claim 2 wherein $R^3$ and $R^4$ are both hydrogen

or together with the nitrogen to which they are attached form a pyrrolidinyl, morpholinyl, piperazinyl or 4-methyl-piperazinyl ring.

4. A compound of formula I as claimed in any one of Claims 1 to 3 wherein $R^5$ is hydrogen and $R^6$ is hydrogen or methyl.

5. A compound of the formula:

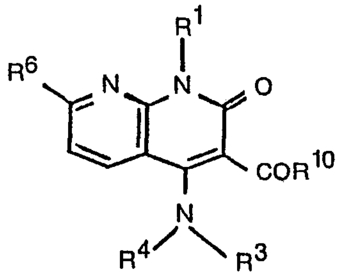

(II)

in which

$R^1$ is hydrogen, methyl, ethyl, propyl, allyl, propargyl, cyclohexyl, or isobutyl;

$R^3$ is hydrogen, alkyl of 1 to 6 carbon atoms or trifluoroacetyl;

$R^4$ is hydrogen, alkyl of 1 to 6 carbon atoms, or when taken with $R^3$ and the nitrogen atom to which they are attached, forms a pyrrolidinyl, morpholinyl, piperazinyl or 4-methylpiperazinyl ring;

$R^6$ is hydrogen or methyl;

$R^{10}$ is hydroxy, alkoxy of 1 to 6 carbon atoms or hydrazinyl; or a pharmaceutically acceptable salt thereof.

6. A 4-amino-1-alkyl-1,2-dihydro-2-oxo-1,8-naphthyridine as claimed in Claim 1 which is: 4-amino-7-chloro-1-ethyl-1,2-dihydro-2-oxo-1,8-naphthyridine-3-carboxylic acid ethyl ester, or
4-amino-7-ethoxy-1-ethyl-1,2-dihydro-2-oxo-1,8-naphthyridine-3-carboxylic acid ethyl ester, or
4-amino-7-ethylamino-1,N-diethyl-1,2-dihydro-2-oxo-1,8-naphthyridine-3-carboxamide, or
4-amino-1-ethyl-1,2-dihydro-7-methyl-2-oxo-1,8-naphthyridine-3-carboxylic acid ethyl ester, or
4-amino-1-ethyl-1,2-dihydro-7-methyl-2-oxo-1,8-naphthyridine-3-carbonitrile, or
4-amino-1-ethyl-1,2-dihydro-7-methyl-2-oxo-1,8-naphthyridine-3-carboxamide, or
4-amino-1-methyl-1,2-dihydro-2-oxo-1,8-naphthyridine-3-carboxylic acid ethyl ester, or
4-amino-1-ethyl-1,2-dihydro-2-oxo-1,8-naphthyridine-3-carboxylic acid, n-butyl ester, or
4-amino-1-ethyl-1,2-dihydro-2-oxo-1,8-naphthyridine-3-carboxylic acid, or
4-amino-1-propyl-1,2-dihydro-2-oxo-1,8-naphthyridine-3-carboxylic acid ethyl ester, or
4-amino-1-isobutyl-1,2-dihydro-2-oxo-1,8-naphthyridine-3-carboxylic acid ethyl ester, or
4-amino-1-cyclohexyl-1,2-dihydro-2-oxo-1,8-naphthyridine-3-carboxylic acid ethyl ester; or a pharmaceutically acceptable salt thereof.

7. A 4-amino-1,2-dihydro-2-oxo-1,8-naphthyridine as claimed in Claim 1 which is:
4-amino-1,2-dihydro-2-oxo-1,8-naphthyridine-3-carboxylic acid ethyl ester, or
4-amino-1,2-dihydro-2-oxo-1,8-naphthyridine-3-carboxylic acid; or a pharmaceutically acceptable salt thereof.

8. 1-Allyl-4-amino-1,2-dihydro-2-oxo-1,8-naphthyridine-3-carboxylic acid ethyl ester or a pharmaceutically acceptable salt thereof.

9. 1-Propargyl-4-amino-2-oxo-1,8-naphthyridine-3-carboxylic acid ethyl ester, or a pharmaceutically acceptable salt thereof.

10. A 4-pyrrolidinyl-1-alkyl-1,2-dihydro-2-oxo-1,8-naphthyridine as claimed in Claim 1 which is:
1-methyl-4-(1-pyrrolidinyl)-1,2-dihydro-2-oxo-1,8-naphthyridine-3-carboxylic acid ethyl ester, or
1,7-dimethyl-4-(1-pyrrolidinyl)-1,2-dihydro-2-oxo-1,8-naphthyridine-3-carboxylic acid ethyl ester, or
1-ethyl-1,2-dihydro-7-methyl-2-oxo-4-(1-pyrrolidinyl)-1,8-naphthyridine-3-carboxamide, or
1-ethyl-1,2-dihydro-7-methyl-2-oxo-4-(1-pyrrolidinyl)-1,8-naphthyridine-3-carboxylic acid ethyl ester; or a pharmaceutically acceptable salt thereof.

11. 1-Allyl-1,2-dihydro-7-methyl-2-oxo-4-(1-pyrrolidinyl)-1,8-naphthyridine-3-carboxylic acid ethyl ester or a pharmaceutically acceptable salt thereof.

12. A 4-(4-substituted piperazinyl)-1,2-dihydro-2-oxo-1,8-naphthyridine as claimed in Claim 1 which is 1-methyl-4-(4-methyl-1-piperazinyl)-1,2-dihydro-2-oxo-1,8-naphthyridine-3-carboxylic acid ethyl ester, or
1,7-dimethyl-4-(4-methyl-1-piperazinyl)-1,2-dihydro-2-oxo-1,8-naphthyridine-3-carboxylic acid ethyl ester, or
1-ethyl-1,2-dihydro-4-(4-methyl-1-piperazinyl)-2-oxo-1,8-naphthyridine-3-carboxylic acid ethyl ester, or
1-ethyl-1,2-dihydro-7-methyl-2-oxo-4-(4-phenyl-1-piperazinyl)-1,8-naphthyridine-3-carboxylic acid ethyl ester, or
4,4′-(1,4-piperazinediyl)-bis[1-ethyl-1,2-dihydro-7-methyl-2-oxo-1,8-naphthyridine-3-carboxylic acid]-diethyl ester, or

1-ethyl-1,2-dihydro-4-(4-carbethoxy-1-piperazinyl)-7-methyl-2-oxo-1,8-naphthyridine-3-carboxylic acid, ethyl ester; or a pharmaceutically acceptable salt thereof.

13. A 4-alkyl- or substituted alkyl -amino-1,2-dihydro-2-oxo-1,8-naphthyridine as claimed in Claim 1 which is 4-diethylamino-1-ethyl-1,2-dihydro-7-methyl-2-oxo-1,8-naphthyridine-3-carboxylic acid ethyl ester, or

1-ethyl-1,2-dihydro-4-ethylamino-7-methyl-2-oxo-1,8-naphthyridine-3-carboxylic acid ethyl ester, or

1-ethyl-1,2-dihydro-4-[2-(hydroxyethyl]methyl-amino]-7-methyl-2-oxo-1,8-naphthyridine-3-carboxylic acid ethyl ester, or

1-ethyl-1,2-dihydro-4-(3-dimethylaminopropylamino)-7-methyl-2-oxo-1,8-naphthyridine-3-carboxylic acid ethyl ester; or a pharmaceutically acceptable salt thereof.

14. A 4-acylamino-1,2-dihydro-2-oxo-1,8-naphthyridine as claimed in Claim 1 which is 4-diacetylamino-1-ethyl-1,2-dihydro-2-oxo-1,8-naphthyridine-3-carboxylic acid ethyl ester, or

1-ethyl-1,2-dihydro-4-trifluoroacetylamino-2-oxo-1,8-naphthyridine-3-carboxylic acid ethyl ester, or

1-Allyl-4-chloroacetylamino-1,2-dihydro-2-oxo-1,8-naphthyridine-3-carboxylic acid; or a pharmaceutically acceptable salt thereof.

15. 1-Ethyl-1,2-dihydro-7-methyl-4-(4-morpholinyl)-2-oxo-1,8-naphthyridine-3-carboxylic acid or a pharmaceutically acceptable salt thereof.

16. A 4-amino-1-ethyl-1,2-dihydro-2-oxo-1,8-naphthyridine as claimed in Claim 1 which is 4-amino-1-ethyl-1,2-dihydro-2-oxo-1,8-naphthyridine-3-carboxylic acid ethyl ester or

4-amino-1-ethyl-1,2-dihydro-2-oxo-1,8-naphthyridine-3-carboxylic acid hydrazide, or a pharmaceutically acceptable salt thereof.

17. A 4-piperazinyl-1-ethyl-1,2-dihydro-2-oxo-1,8-napthyridine as claimed in Claim 1 which is 1-ethyl-1,2-dihydro-7-methyl-2-oxo-4-(4-methyl-1-piperazinyl)-1,8-naphthyridine-3-carboxylic acid or 1-ethyl-1,2-dihydro-7-methyl-4-(4-methyl-1-piperazinyl)-2-oxo-1,8-naphthyridine-3-carboxylic acid ethyl ester or 1-ethyl-1,2-dihydro-7-methyl-2-oxo-4-(1-piperazinyl)-1,8-naphthyridine-3-carboxylic acid ethyl ester, or a pharmaceutically acceptable salt thereof.

18. A process for preparing a compound as claimed in Claim 1 which comprises:

(a) reacting a 2-aminonicotinonitrile of formula

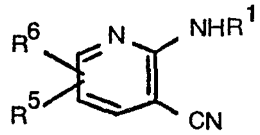

wherein $R^1$, $R^5$ and $R^6$ are as defined in Claim 1, with (i) a lower alkyl malonyl halide followed by ring closure under basic conditions or (ii) an alkali metal di(lower)alkyl malonate ester, to give after acidification a compound of formula I wherein $R^2$ is $CO_2R^7$ and $R^3$ and $R^4$ are both hydrogen, the term "lower" used in connection with alkyl meaning such a group contains 1—6 carbon atoms; or

(b) reacting a compound of formula

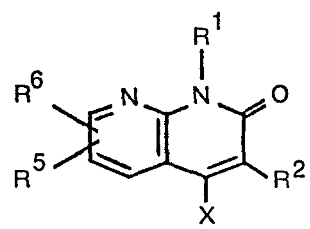

(IV)

wherein $R^1$, $R^5$ and $R^6$ are as defined in Claim 1 and $R^2$ is —CN, $COOR^7$ or —$CONR^8R^9$ wherein $R^7$, $R^8$ and $R^9$ are as defined in Claim 1 and X is chlorine, bromine or iodine with an amine of formula $HNR^3R^4$ wherein $R^3$ and $R^4$ are as defined in Claim 1 to give a corresponding compound of formula I; or

(c) reacting a compound of formula

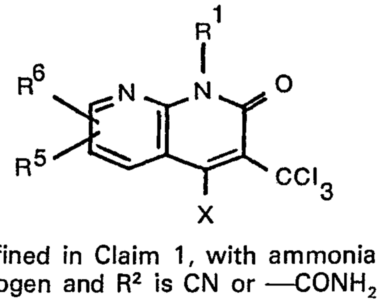

wherein $R^1$, $R^5$ and $R^6$ are as defined in Claim 1, with ammonia to give a compound of formula I, wherein $R^3$ and $R^4$ are both hydrogen and $R^2$ is CN or —$CONH_2$

or (d) alkylating, alkenylating or alkynylating an alkali metal salt of a compound of formula I wherein $R^1$ is an alkali metal to give a compound of formula I wherein $R^1$ is alkyl, alkenyl or alkynyl as defined in Claim 1.

or (e) converting an ester of formula I wherein $R^2$ is $—COOR^7$ as defined in Claim 1 to give an acid of formula I wherein $R^2$ is COOH or a pharmaceutically acceptable salt thereof.

or (f) converting a compound of formula I wherein $R^5$ and/or $R^6$ is halogen to a compound of formula I wherein $R^5$ and/or $R^6$ is lower alkoxy of 1 to 6 carbon atoms or lower alkylamino of 1 to 4 carbon atoms with simultaneous conversion of $R^2$ when $COOR^7$ to the corresponding lower alkylamide,

or (g) reacting a compound of formula I in which $R^2$ is $CO_2R^7$ with hydrazine, ammonia or an amine of formula $NHR^8R^9$ wherein $R^8$ and $R^9$ are as defined in Claim 1 to give a compound of formula I wherein $R^2$ is $—CONHNH_2$, $—CONH_2$ or $—CONR^8R^9$.

or (h) acylating a compound of formula I wherein $R^3$ and $R^4$ are both hydrogen to give a compound of formula I wherein $R^3$ and/or $R^4$ are alkanoyl of 2 to 4 carbon atoms or haloalkanoyl of 2 to 4 carbon atoms,

or (i) converting a compound of formula I as defined in Claim 1 to a pharmaceutically acceptable salt.

19. A compound of formula I as claimed in any one of Claims 1 to 17 for use as pharmaceutical.

20. A pharmaceutical composition comprising a compound of formula I as claimed in any one of Claims 1 to 17 and a pharmaceutically acceptable carrier.

**Claims for the Contracting State: AT**

1. A process for preparing compound of the formula:

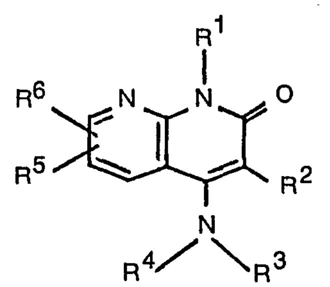

(I)

in which $R^1$ is hydrogen, alkyl of 1 to 6 carbon atoms, cycloalkyl of 5 to 6 carbon atoms, alken-(3,4,5 or 6)-yl of 3 to 6 carbon atoms or alkyn-(3,4,5 or 6)-yl of 3 to 6 carbon atoms; $R^2$ is —CN, $—CO_2H$, $—CO_2R^7$ where $R^7$ is alkyl of 1 to 6 carbon atoms, $—CONHNH_2$ or

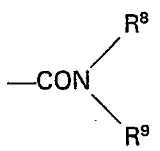

where $R^8$ and $R^9$ are independently hydrogen or alkyl of 1 to 6 carbon atoms; $R^3$ and $R^4$ are independently hydrogen, alkyl of 1 to 6 carbon atoms, hydroxyalkyl of 1 to 6 carbon atoms, dialkylaminoalkyl of 4 to 8 carbon atoms, alkanoyl of 2 to 4 carbon atoms, haloalkanoyl of 2 to 4 carbon atoms, or when taken together with the nitrogen atom to which they are attached, $R^3$ and $R^4$ complete a heterocyclic group selected from aziridinyl, azetidinyl, pyrrolidinyl, piperidinyl, piperazinyl, 4-lower alkylpiperazinyl, 4-phenylpiperazinyl, 4-[1-ethyl-1,2-dihydro-7-methyl-2-oxo-1,8-naphthyridine-4-yl-3-carboxylic acid ethyl ester]piperazinyl, 4-carb(lower)alkoxy-piperazinyl, morpholinyl, thiomorpholinyl or a ring substituted lower alkyl analogue thereof; $R^5$ and $R^6$ are independently hydrogen, alkyl of 1 to 4 carbon atoms, halo, alkylamino of 1 to 4 carbon atoms, or alkoxy of 1 to 6 carbon atoms or a pharmaceutically acceptable salt thereof; the term "lower" used in connection with alkyl or alkoxy groups meaning such groups contain 1 to 6 carbon atoms; which comprises

(a) reacting a 2-aminonicotinonitrile of formula

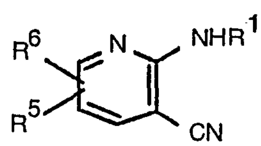

wherein $R^1$, $R^5$ and $R^6$ are as defined above with (i) a lower alkyl malonyl halide followed by ring closure under basic conditions or (ii) an alkali metal di(lower)alkyl malonate ester, wherein lower is as

defined above to give after acidification a compound of formula I wherein $R^2$ is $CO_2R'$ and $R^3$ and $R^4$ are both hydrogen, or

(b) reacting a compound of formula

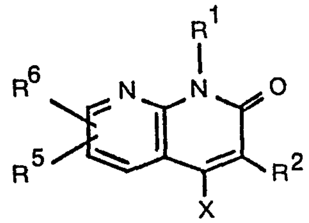

(IV)

wherein $R^1$, $R^5$ and $R^6$ are as defined above and $R^2$ is —CN, $COOR^7$ or —$CONR^8R^9$ wherein $R^7$, $R^8$ and $R^9$ are as defined above and X is chlorine, bromine or iodine with an amine of formula $HNR^3R^4$ wherein $R^3$ and $R^4$ are as defined above to give a corresponding compound of formula I; or

(c) reacting a compound of formula

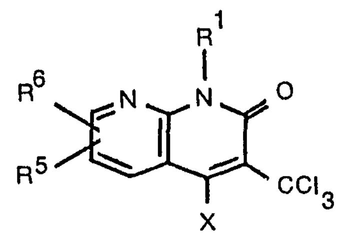

wherein $R^1$, $R^5$ and $R^6$ are as defined above, with ammonia to give a compound of formula I, wherein $R^3$ and $R^4$ are both hydrogen and $R^2$ is CN or —$CONH_2$

or (d) alkylating, alkenylating or alkynylating an alkali metal salt of a compound of formula I wherein $R^1$ is an alkali metal to give a compound of formula I wherein $R^1$ is alkyl, alkenyl or alkynyl as defined above;

or (e) converting an ester of formula I wherein $R^2$ is —$COOR^7$ as defined above to give an acid of formula I wherein $R^2$ is COOH or a pharmaceutically acceptable salt thereof;

or (f) converting a compound of formula I wherein $R^5$ and/or $R^6$ is halogen to a compound of formula I wherein $R^5$ and/or $R^6$ is lower alkoxy of 1 to 6 carbon atoms or lower alkylamino of 1 to 4 carbon atoms with simultaneous conversion of $R^2$ when $COOR^7$ to the corresponding lower alkylamide,

or (g) reacting a compound of formula I in which $R^2$ is $CO_2R^7$ with hydrazine, ammonia or an amine of formula $HNR^8R^9$ wherein $R^8$ and $R^9$ are as defined above to give a compound of formula I wherein $R^2$ is —$CONHNH_2$, —$CONH_2$ or —$CONR^8R^9$,

or (h) acylating a compound of formula I wherein $R^3$ and $R^4$ are both hydrogen to give a compound of formula I wherein $R^3$ and/or $R^4$ are alkanoyl of 2 to 4 carbon atoms or haloalkanoyl of 2 to 4 carbon atoms,

or (i) converting a compound of formula I as defined above to a pharmaceutically acceptable salt.

2. A process as claimed in Claim 1 wherein, in the product, $R^1$ and $R^7$ are alkyl of 1 to 4 carbon atoms.

3. A process as claimed in Claim 1 or Claim 2, wherein, in the product, $R^3$ and $R^4$ are both hydrogen or together with the nitrogen to which they are attached form a pyrrolidinyl, morpholinyl, piperazinyl or 4-methylpiperazinyl ring.

4. A process as claimed in any one of Claims 1 to 3 wherein, in the product, $R^5$ is hydrogen and $R^6$ is hydrogen or methyl.

5. A process as claimed in Claim 1 in which the product is a compound of the formula;

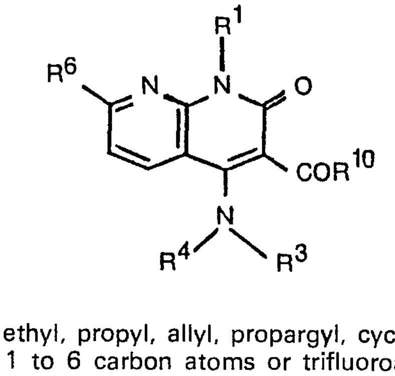

(II)

in which

$R^1$ is hydrogen, methyl, ethyl, propyl, allyl, propargyl, cyclohexyl, or isobutyl;

$R^3$ is hydrogen, alkyl of 1 to 6 carbon atoms or trifluoroacetyl;

$R^4$ is hydrogen, alkyl of 1 to 6 carbon atoms, or when taken with $R^3$ and the nitrogen atom to

which they are attached, forms a pyrrolidinyl, morpholinyl, piperazinyl or 4-methylpiperazinyl ring;

$R^6$ is hydrogen or methyl;

$R^{10}$ is hydroxy, alkoxy of 1 to 6 carbon atoms or hydrazinyl; or a pharmaceutically acceptable salt thereof.

6. A process as claimed in Claim 1 for preparing a 4-amino-1-alkyl-1,2-dihydro-2-oxo-1,8-naphthyridine in which the product is:

4-amino-7-chloro-1-ethyl-1,2-dihydro-2-oxo-1,8-naphthyridine-3-carboxylic acid ethyl ester, or
4-amino-7-ethoxy-1-ethyl-1,2-dihydro-2-oxo-1,8-naphthyridine-3-carboxylic acid ethyl ester, or
4-amino-7-ethylamino-1,N-diethyl-1,2-dihydro-2-oxo-1,8-naphthyridine-3-carboxamide, or
4-amino-1-ethyl-1,2-dihydro-7-methyl-2-oxo-1,8-naphthyridine-3-carboxylic acid ethyl ester, or
4-amino-1-ethyl-1,2-dihydro-7-methyl-2-oxo-1,8-naphthyridine-3-carbonitrile, or
4-amino-1-ethyl-1,2-dihydro-7-methyl-2-oxo-1,8-naphthyridine-3-carboxamide, or
4-amino-1-methyl-1,2-dihydro-2-oxo-1,8-naphthyridine-3-carboxylic acid ethyl ester, or
4-amino-1-ethyl-1,2-dihydro-2-oxo-1,8-naphthyridine-3-carboxylic acid, n-butyl ester, or
4-amino-1-ethyl-1,2-dihydro-2-oxo-1,8-naphthyridine-3-carboxylic acid, or
4-amino-1-propyl-1,2-dihydro-2-oxo-1,8-naphthyridine-3-carboxylic acid ethyl ester, or
4-amino-1-isobutyl-1,2-dihydro-2-oxo-1,8-naphthyridine-3-carboxylic acid ethyl ester, or
4-amino-1-cyclohexyl-1,2-dihydro-2-oxo-1,8-naphthyridine-3-carboxylic acid ethyl ester; or a pharmaceutically acceptable salt thereof.

7. A process as claimed in Claim 1 for preparing a 4-amino-1,2-dihydro-2-oxo-1,8-naphthyridine in which the product is:

4-amino-1,2-dihydro-2-oxo-1,8-naphthyridine-3-carboxylic acid ethyl ester, or
4-amino-1,2-dihydro-2-oxo-1,8-naphthyridine-3-carboxylic acid; or a pharmaceutically acceptable salt thereof.

8. A process as claimed in Claim 1 in which the product is:

1-allyl-4-amino-1,2-dihydro-2-oxo-1,8-naphthyridine-3-carboxylic acid ethyl ester or a pharmaceutically acceptable salt thereof.

9. A process as claimed in Claim 1 in which the product is:

1-propargyl-4-amino-2-oxo-1,8-naphthyridine-3-carboxylic acid ethyl ester, or a pharmaceutically acceptable salt thereof.

10. A process as claimed in Claim 1 for preparing a 4-pyrrolidinyl-1-alkyl-1,2-dihydro-2-oxo-1,8-naphthyridine in which the product is:

1-methyl-4-(1-pyrrolidinyl)-1,2-dihydro-2-oxo-1,8-naphthyridine-3-carboxylic acid ethyl ester, or
1,7-dimethyl-4-(1-pyrrolidinyl)-1,2-dihydro-2-oxo-1,8-naphthyridine-3-carboxylic acid ethyl ester, or
1-ethyl-1,2-dihydro-7-methyl-2-oxo-4-(1-pyrrolidinyl)-1,8-naphthyridine-3-carboxamide, or
1-ethyl-1,2-dihydro-7-methyl-2-oxo-4-(1-pyrrolidinyl)-1,8-naphthyridine-3-carboxylic acid ethyl ester; or a pharmaceutically acceptable salt thereof.

11. A process as claimed in Claim 1 in which the product is:

1-allyl-1,2-dihydro-7-methyl-2-oxo-4-(1-pyrrolidinyl)-1,8-naphthyridine-3-carboxylic acid ethyl ester or a pharmaceutically acceptable salt thereof.

12. A process as claimed in Claim 1 for preparing a 4-(4-substituted piperazinyl)-1,2-dihydro-2-oxo-1,8-naphthyridine in which the product is:

1-methyl-4-(4-methyl-1-piperazinyl)-1,2-dihydro-2-oxo-1,8-naphthyridine-3-carboxylic acid ethyl ester, or
1,7-dimethyl-4-(4-methyl-1-piperazinyl)-1,2-dihydro-2-oxo-1,8-naphthyridine-3-carboxylic acid ethyl ester, or
1-ethyl-1,2-dihydro-4-(4-methyl-1-piperazinyl)-2-oxo-1,8-naphthyridine-3-carboxylic acid ethyl ester, or
1-ethyl-1,2-dihydro-7-methyl-2-oxo-4-(4-phenyl-1-piperazinyl)-1,8-naphthyridine-3-carboxylic acid ethyl ester, or
4,4′-(1,4-piperazinediyl)-bis[1-ethyl-1,2-dihydro-7-methyl-2-oxo-1,8-naphthyridine-3-carboxylic acid]-diethyl ester, or
1-ethyl-1,2-dihydro-4-(4-carbethoxy-1-piperazinyl)-7-methyl-2-oxo-1,8-naphthyridine-3-carboxylic acid, ethyl ester; or a pharmaceutically acceptable salt thereof.

13. A process as claimed in Claim 1 for preparing a 4-alkyl- or substituted alkyl-amino-1,2-dihydro-2-oxo-1,8-naphthyridine in which the product is:

4-diethylamino-1-ethyl-1,2-dihydro-7-methyl-2-oxo-1,8-naphthyridine-3-carboxylic acid ethyl ester, or
1-ethyl-1,2-dihydro-4-ethylamino-7-methyl-2-oxo-1,8-naphthyridine-3-carboxylic acid ethyl ester, or
1-ethyl-1,2-dihydro-4-[2-(hydroxyethylmethylamino]-7-methyl-2-oxo-1,8-naphthyridine-3-carboxylic acid ethyl ester, or
1-ethyl-1,2-dihydro-4-(3-dimethylaminopropylamino)-7-methyl-2-oxo-1,8-naphthyridine-3-carboxylic acid ethyl ester; or a pharmaceutically acceptable salt thereof.

14. A process as claimed in Claim 1 for preparing a 4-acylamino-1,2-dihydro-2-oxo-1,8-naphthyridine in which the product is:

4-diacetylamino-1-ethyl-1,2-dihydro-2-oxo-1,8-naphthyridine-3-carboxylic acid ethyl ester, or

1-ethyl-1,2-dihydro-4-trifluoroacetylamino-2-oxo-1,8-naphthyridine-3-carboxylic acid ethyl ester, or

1-allyl-4-chloroacetylamino-1,2-dihydro-2-oxo-1,8-naphthyridine-3-carboxylic acid; or a pharmaceutically acceptable salt thereof.

15. A process as claimed in Claim 1 in which the product is:

1-ethyl-1,2-dihydro-7-methyl-4-(4-morpholinyl)-2-oxo-1,8-naphthyridine-3-carboxylic acid or a pharmaceutically acceptable salt thereof.

16. A process as claimed in Claim 1 for preparing a 4-amino-1-ethyl-1,2-dihydro-2-oxo-1,8-naphthyridine in which the product is:

4-amino-1-ethyl-1,2-dihydro-2-oxo-1,8-naphthyridine-3-carboxylic acid ethyl ester or

4-amino-1-ethyl-1,2-dihydro-2-oxo-1,8-naphthyridine-3-carboxylic acid hydrazide, or a pharmaceutically acceptable salt thereof.

17. A process as claimed in Claim 1 for preparing a 4-piperazinyl-1-ethyl-1,2-dihydro-2-oxo-1,8-naphthyridine in which the product is:

1-ethyl-1,2-dihydro-7-methyl-2-oxo-4-(4-methyl-1-piperazinyl)-1,8-naphthyridine-3-carboxylic acid or

1-ethyl-1,2-dihydro-7-methyl-4-(4-methyl-1-piperazinyl)-2-oxo-1,8-naphthyridine-3-carboxylic acid ethyl ester or 1-ethyl-1,2-dihydro-7-methyl-2-oxo-4-(1-piperazinyl)-1,8-naphthyridine-3-carboxylic acid ethyl ester, or a pharmaceutically acceptable salt thereof.

18. A process for producing a therapeutic composition characterised in that a compound of formula I as defined in Claim 1 or a pharmaceutically acceptable salt thereof and a pharmaceutically acceptable carrier are brought into a form suitable for therapeutic administration.

**Revendications pour les Etats contractants: BE CH LI DE FR IT LU NL SE**

1. Composé de formule:

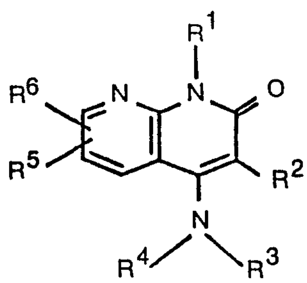

(I)

dans laquelle $R^1$ est l'hydrogène, un groupe alkyle ayant 1 à 6 atomes de carbone, un groupe cycloalkyle ayant 5 à 6 atomes de carbone, un groupe alcène-(3,4,5 ou 6)-yle ayant 3 à 6 atomes de carbone ou un groupe alcyne-(3,4,5 ou 6)-yle ayant 3 à 6 atome de carbone; $R^2$ est un groupe $-CN$, $-CO_2H$, $-CO_2R^7$ où $R^7$ est un radical alkyle ayant 1 à 6 atomes de carbone, un groupe $-CONHNH_2$ ou un groupe

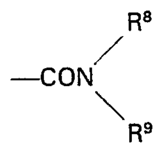

dans lequel $R^8$ et $R^9$ représentent, indépendamment, l'hydrogène ou un radical alkyle ayant 1 à 6 atomes de carbone; $R^3$ et $R^4$ sont, indépendamment, l'hydrogène, un radical alkyle ayant 1 à 6 atomes de carbone, un radical hydroxyalkyle ayant 1 à 6 atomes de carbone, un radical dialkylaminoalkyle ayant 4 à 8 atomes de carbone, un radical alcanoyle ayant 2 à 4 atomes de carbone, un radical halogénalcanoyle ayant 2 à 4 atomes de carbone, ou bien $R^3$ et $R^4$ forment, conjointement avec l'atome d'azote auquel ils sont liés, un groupe hétérocyclique choisi entre des groupes aziridinyle, azétidinyle, pyrrolidinyle, pipéridinyle, pipérazinyle, 4-(alkyle inférieur)pipérazinyle, 4-phénylpipérazinyle, 4-[1-éthyl-1,2-dihydro-7-méthyl-2-oxo-1,8-naphtyridine-4-yl-3-carboxylate d'éthyle]pipérazinyle, 4-carb(alkoxy inférieur)-pipérazinyle, morpholinyle, thiomorpholinyle ou un analogue alkylique inférieur substitué sur le noyau; $R^5$ et $R^6$ représentent, indépendamment, l'hydrogène, un groupe alkyle ayant 1 à 4 atomes de carbone, halogéno, alkylamino ayant 1 à 4 atomes de carbone ou un groupe alkoxy ayant 1 à 6 atomes de carbone, ou un sel pharmaceutiquement acceptable de ce composé; le terme "inférieur" utilisé pour qualifier un groupe alkyle ou alkoxy signifiant que ces groupes contiennent 1 à 6 atomes de carbone.

2. Composé de formule I suivant la revendication 1, dans lequel $R^1$ et $R^7$ sont des groupes alkyle ayant 1 à 4 atomes de carbone.

3. Composé de formule I suivant la revendication 1 ou la revendication 2, dans lequel $R^3$ et $R^4$ sont tous deux de l'hydrogène ou forment, conjointement avec l'atome d'azote auquel ils sont liés, un noyau pyrrolidinyle, morpholinyle, pipérazinyle ou 4-méthylpipérazinyle.

4. Composé de formule I suivant l'une quelconque des revendications 1 à 3, dans lequel $R^5$ est l'hydrogène et $R^6$ est l'hydrogène ou le groupe méthyle.

5. Composé de formule:

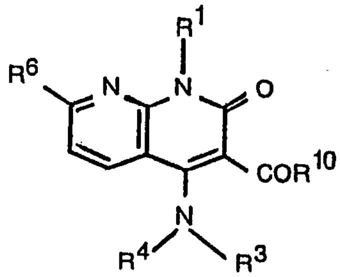

(II)

dans laquelle

$R^1$ est l'hydrogène ou le groupe méthyle, éthyle, propyle, allyle, propargyle, cyclohexyle ou isobutyle;

$R^3$ est l'hydrogène, un groupe alkyle ayant 1 à 6 atomes de carbone ou le groupe trifluoracétyle;

$R^4$ est l'hydrogène, un groupe alkyle ayant 1 à 6 atomes de carbone, ou forme avec $R^3$ et l'atome d'azote auquel ils sont liés, un noyau pyrrolidinyle, morpholinyle, pipérazinyle ou 4-méthylpipérazinyle;

$R^6$ est l'hydrogène ou le groupe méthyle;

$R^{10}$ est un groupe hydroxy, alkoxy ayant 1 à 6 atomes de carbone ou hydrazinyle; ou un sel pharmaceutiquement acceptable de ce composé.

6. Une 4-amino-1-alkyl-1,2-dihydro-2-oxo-1,8-naphtyridine suivant la revendication 1, qui est;

l'ester éthylique de l'acide 4-amino-7-chloro-1-éthyl-1,2-dihydro-2-oxo-1,8-naphtyridine-3-carboxylique, ou

l'ester éthylique de l'acide 4-amino-7-éthoxy-1-éthyl-1,2-dihydro-2-oxo-1,8-naphtyridine-3-carboxylique, ou

le 4-amino-7-éthylamino-1,N-diéthyl-1,2-dihydro-2-oxo-1,8-naphtyridine-3-carboxamide, ou

l'ester éthylique de l'acide 4-amino-1-éthyl-1,2-dihydro-7-méthyl-2-oxo-1,8-naphtyridine-3-carboxylique, ou

le 4-amino-1-éthyl-1,2-dihydro-7-méthyl-2-oxo-1,8-naphtyridine-3-carbonitrile, ou

le 4-amino-1-éthyl-1,2-dihydro-7-méthyl-2-oxo-1,8-naphtyridine-3-carboxamide, ou

l'ester éthylique de l'acide 4-amino-1-méthyl-1,2-dihydro-2-oxo-1,8-naphtyridine-3-carboxylique, ou

l'ester n-butylique de l'acide 4-amino-1-éthyl-1,2-dihydro-2-oxo-1,8-naphtyridine-3-carboxylique, ou

l'acide 4-amino-1-éthyl-1,2-dihydro-2-oxo-1,8-naphtyridine-3-carboxylique, ou

l'ester éthylique de l'acide 4-amino-1-propyl-1,2-dihydro-2-oxo-1,8-naphtyridine-3-carboxylique, ou

l'ester éthylique de l'acide 4-amino-1-isobutyl-1,2-dihydro-2-oxo-1,8-naphtyridine-3-carboxylique, ou

l'ester éthylique de l'acide 4-amino-1-cyclohexyl-1,2-dihydro-2-oxo-1,8-naphtyridine-3-carboxylique; ou un sel pharmaceutiquement acceptable de ce composé.

7. 4-amino-1,2-dihydro-2-oxo-1,8-naphtyridine suivant la revendication 1 qui est:

l'ester éthylique de l'acide 4-amino-1,2-dihydro-2-oxo-1,8-naphtyridine-3-carboxylique, ou

l'acide 4-amino-1,2-dihydro-2-oxo-1,8-naphtyridine-3-carboxylique; ou un sel pharmaceutiquement acceptable de ce composé.

8. L'ester éthylique de l'acide 1-allyl-4-amino-1,2-dihydro-2-oxo-1,8-naphtyridine-3-carboxylique ou un sel pharmaceutiquement acceptable de ce composé.

9. L'ester éthylique de l'acide 1-propargyl-4-amino-2-oxo-1,8-naphtyridine-3-carboxylique ou un sel pharmaceutiquement acceptable de ce composé.

10. 4-pyrrolidinyl-1-alkyl-1,2-dihydro-2-oxo-1,8-naphtyridine suivant la revendication 1, qui est:

l'ester éthylique de l'acide 1-méthyl-4-(1-pyrrolidinyl)-1,2-dihydro-2-oxo-1,8-naphtyridine-3-carboxylique, ou l'ester éthylique de l'acide 1,7-diméthyl-4-(1-pyrrolidinyl)-1,2-dihydro-2-oxo-1,8-naphtyridine-3-carboxylique, ou

le 1-éthyl-1,2-dihydro-7-méthyl-2-oxo-4-(1-pyrrolidinyl)-1,8-naphtyridine-3-carboxamide, ou

l'ester éthylique de l'acide 1-éthyl-1,2-dihydro-7-méthyl-2-oxo-4-(1-pyrrolidinyl)-1,8-naphtyridine-3-carboxylique; ou un sel pharmaceutiquement acceptable de ce composé.

11. L'ester éthylique de l'acide 1-allyl-1,2-dihydro-7-méthyl-2-oxo-4-(1-pyrrolidinyl)-1,8-naphtyridine-3-carboxylique ou un sel pharmaceutiquement acceptable de ce composé.

12. 4-(pipérazinyl substitué en position 4)-1,2-dihydro-2-oxo-1,8-naphtyridine suivant la revendication 1, qui est

l'ester éthylqie de l'acide 1-méthyl-4-(4-méthyl-1-pipérazinyl)-1,2-dihydro-2-oxo-1,8-naphtyridine-3-carboxylique, ou

l'ester éthylique de l'acide 1,7-diméthyl-4-(4-méthyl-1-pipérazinyl)-1,2-dihydro-2-oxo-1,8-naphtyridine-3-carboxylique, ou

l'ester éthylique de l'acide 1-éthyl-1,2-dihydro-4-(4-méthyl-1-pipérazinyl)-2-oxo-1,8-naphtyridine-3-carboxylique, ou

l'ester éthylique de l'acide 1-éthyl-1,2-dihydro-7-méthyl-2-oxo-4-(4-phényl-1-pipérazinyl)-1,8-naphtyridine-3-carboxylique, ou

l'ester diéthylique de l'acide 4,4'-(1,4-pipérazinediyl)-bis[1-éthyl-1,2-dihydro-7-méthyl-2-oxo-1,8-naphtyridine-3-carboxylique], ou

l'ester éthylique de l'acide 1-éthyl-1,2-dihydro-4-(4-carbéthoxy-1-pipérazinyl)-7-méthyl-2-oxo-1,8-naphtyridine-3-carboxylique, ou un sel pharmaceutiquement acceptable de ce composé.

13. 4-alkyl- ou -(alkyle substitué)-amino-1,2-dihydro-2-oxo-1,8-naphtyridine suivant la revendication 1 qui est

l'ester éthylique de l'acide 4-diéthylamino-1-éthyl-1,2-dihydro-7-méthyl-2-oxo-1,8-naphtyridine-3-carboxylique, ou

l'ester éthylique de l'acide 1-éthyl-1,2-dihydro-4-éthyl-amino-7-méthyl-2-oxo-1,8-naphtyridine-3-carboxylique, ou

l'ester éthylique de l'acide 1-éthyl-1,2-dihydro-4-[2-(hydroxyéthyl)méthylamino]-7-méthyl-2-oxo-1,8-naphtyridine-3-carboxylique, ou

l'ester éthylique de l'acide 1-éthyl-1,2-dihydro-4-(3-diméthylaminopropylamino)-7-méthyl-2-oxo-1,8-naphtyridine-3-carboxylique; ou un sel pharmaceutiquement acceptable de ce composé.

14. 4-acylamino-1,2-dihydro-2-oxo-1,8-naphtyridine suivant la revendication 1, qui est l'ester éthylique de l'acide 4-diacétylamino-1-éthyl-1,2-dihydro-2-oxo-1,8-naphtyridine-3-carboxylique, ou

l'ester éthylique de l'acide 1-éthyl-1,2-dihydro-4-trifluoracétylamino-2-oxo-1,8-naphtyridine-3-carboxylique, ou

l'acide 1-allyl-4-chloroacétylamino-1,2-dihydro-2-oxo-1,8-naphtyridine-3-carboxylique; ou un sel pharmaceutiquement acceptable de ce composé.

15. L'acide 1-éthyl-1,2-dihydro-7-méthyl-4-(4-morpholinyl)-2-oxo-1,8-naphtyridine-3-carboxylique, ou un sel pharmaceutiquement acceptable de ce composé.

16. 4-amino-1-éthyl-1,2-dihydro-2-oxo-1,8-naphtyridine suivant la revendication 1, qui est l'ester éthylique de l'acide 4-amino-1-éthyl-1,2-dihydro-2-oxo-1,8-naphtyridine-3-carboxylique, ou

l'hydrazide de l'acide 4-amino-1-éthyl-1,2-dihydro-2-oxo-1,8-naphtyridine-3-carboxylique, ou un sel pharmaceutiquement acceptable de ce composé.

17. 4-pipérazinyl-1-éthyl-1,2-dihydro-2-oxo-1,8-naphtyridine suivant la revendication 1, qui est l'acide 1-éthyl-1,2-dihydro-7-méthyl-2-oxo-4-(4-méthyl-1-pipérazinyl)-1,8-naphtyridine-3-carboxylique ou l'ester éthylique de l'acide 1-éthyl-1,2-dihydro-7-méthyl-4-(4-méthyl-1-pipérazinyl)-2-oxo-1,8-naphtyridine-3-carboxylique ou l'ester éthylique de l'acide 1-éthyl-1,2-dihydro-7-méthyl-2-oxo-4-(1-pipérazinyl)-1,8-naphtyridine-3-carboxylique ou un sel pharmaceutiquement acceptable de ce composé.

18. Procédé de préparation d'un composé suivant la revendication 1, qui comprend:

(a) la réaction d'un 2-aminonicotinonitrile de formule

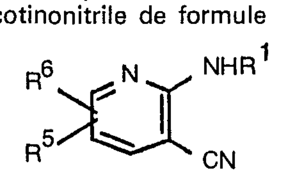

dans laquelle $R^1$, $R^5$ et $R^6$ ont la définition donnée dans la revendication 1, avec (i) un halogénure de (alkyle inférieur)-malonyle, suivie d'une cyclisation dans des conditions basiques, ou (ii) un sel de métal alcalin de malonate de di(alkyle inférieur) pour obtenir après acidification un composé de formule I dans laquelle $R^2$ est le groupe $CO_2R^7$ et $R^3$ et $R^4$ représentent chacun de l'hydrogène, le terme "inférieur" utilisé pour qualifier un groupe alkyle signifiant que ce groupe contient 1 à 6 atomes de carbone; ou

(b) la réaction d'un composé de formule

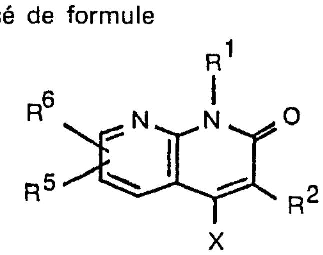

IV

dans laquelle $R^1$, $R^5$ et $R^6$ ont la définition donnée dans la revendication 1 et $R^2$ est un groupe —CN,

COOR[7] ou —CONR[8]R[9] où R[7], R[8] et R[9] ont les définitions données dans la revendication 1 et X est le chlore, le brome ou l'iode, avec une amine de formule $HNR^3R^4$ dans laquelle $R^3$ et $R^4$ ont la définition donnée dans la revendication 1, pour former un composé correspondant de formule I; ou

(c) la réaction d'un composé de formule

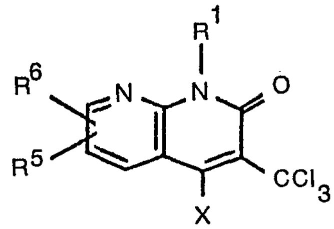

dans laquelle $R^1$, $R^5$ et $R^6$ ont la définition donnée dans la revendication 1, avec l'ammoniac pour obtenir un composé de formule I, dans laquelle $R^3$ et $R^4$ sont tous deux de l'hydrogène et $R^2$ est le groupe CN ou $—CONH_2$ ou

(d) l'alkylation, l'alcénylation ou l'alcynylation d'un sel de métal alcalin d'un composé de formule I dans laquelle $R^1$ est un métal alcalin pour obtenir un composé de formule I dans laquelle $R^1$ est un groupe alkyle, alcényle ou alcynyle comme défini dans la revendication 1 ou

(e) la transformation d'un ester de formule I dans laquelle $R^2$ est un groupe $—COOR^7$ comme défini dans la revendication 1 pour obtenir un acide de formule I dans laquelle $R^2$ est le groupe COOH, ou un sel pharmaceutiquement acceptable de ce composé, ou

(f) la transformation d'un composé de formule I dans laquelle $R^5$ et/ou $R^6$ représentent un halogène, en un composé de formule I dans laquelle $R^5$ et/ou $R^6$ représentent un groupe alkoxy inférieur ayant 1 à 6 atomes de carbone ou un groupe (alkyle inférieur)amino ayant 1 à 4 atomes de carbone avec transformation simultanée de $R^2$, lorsqu'il s'agit de $COOR^7$, en l'alkylamide inférieur correspondant, ou

(g) la réaction d'un composé de formule I dans laquelle $R^2$ est un groupe $CO_2R^7$ avec l'hydrazine, l'ammoniac ou une amine de formule $HNR^8R^9$ dans laquelle $R^8$ et $R^9$ ont la définition donnée dans la revendication 1 pour obtenir un composé de formule I dans laquelle $R^2$ est un groupe $—CONHNH_2$, $—CONH_2$ ou $—CONR^8R^9$, ou

(h) l'acylation d'un composé de formule I dans laquelle $R^3$ et $R^4$ sont tous deux de l'hydrogène pour obtenir un composé de formule I dans laquelle $R^3$ et/ou $R^4$ sont des groupes alcanoyle ayant 2 à 4 atomes de carbone ou halogénalcanoyle ayant 2 à 4 atomes de carbone, ou

(i) la transformation d'un composé de formule I comme défini dans la revendication 1 en un sel pharmaceutiquement acceptable.

19. Composé de formule I suivant l'une quelconque des revendications 1 à 17, destiné à être utilisé comme produit pharmaceutique.

20. Compositions pharmaceutique, comprenant un composé de formule I suivant l'une quelconque des revendications 1 à 17 et un support pharmaceutiquement acceptable.

**Revendications pour l'Etat contractant: AT**

1. Procédé de préparation d'un composé de formule:

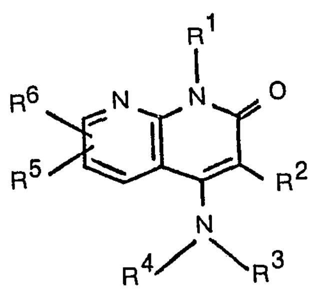

(I)

dans laquelle $R^1$ est l'hydrogène, un groupe alkyle ayant 1 à 6 atomes de carbone, un groupe cycloalkyle ayant 5 à 6 atomes de carbone, un groupe alcène-(3,4,5 ou 6)-yle ayant 3 à 6 atomes de carbone ou un groupe alcyne-(3,4,5 ou 6)-yle ayant 3 à 6 atomes de carbone; $R^2$ est un groupe —CN, $—CO_2H$, $—CO_2R^7$ où $R^7$ est un radical alkyle ayant 1 à 6 atomes de carbone, un groupe $—CONHNH_2$ ou un groupe

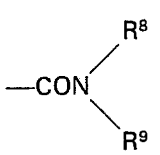

dans lequel $R^8$ et $R^9$ représentent, indépendamment, l'hydrogène ou un radical alkyle ayant 1 à 6 atomes de carbone; $R^3$ et $R^4$ sont, indépendamment, l'hydrogène, un radical alkyle ayant 1 à 6 atomes de carbone, un radical hydroxyalkyle ayant 1 à t atomes de carbone, un radical dialkylaminoalkyle ayant 4 à 8 atomes de carbone, un radical alcanoyle ayant 2 à 4 atomes de carbone, un radical halogénalcanoyle ayant 2 à 4 atomes de carbone, ou bien $R^3$ et $R^4$ forment, conjointement avec l'atome d'azote auquel ils sont liés, un groupe hétérocyclique choisi entre des groupes aziridinyle, azétidinyle, pyrrolidinyle, pipéridinyle, pipérazinyle, 4-(alkyle inférieur)pipérazinyle, 4-phénylpipérazinyle, 4-[1-éthyl-1,2-dihydro-7-méthyl-2-oxo-1,8-naphtyridine-4-yl-3-carboxylate d'éthyle]piérazinyle, 4-carb(alkoxy inférieur)-pipérazinyle, morpholinyle, thiomorpholinyle ou un analogue alkylique inférieur substitué sur le noyau; $R^5$ et $R^6$ représentent, indépendamment, l'hydrogène, un groupe alkyle ayant 1 à 4 atomes de carbone, halogéno, alkylamino ayant 1 à 4 atomes de carbone ou un groupe alkoxy ayant 1 à 6 atomes de carbone, ou un sel pharmaceutiquement acceptable de ce composé; le terme "inférieur utilisé pour qualifier un groupe alkyle ou alkoxy signifiant que ces groupes contiennent 1 à 6 atomes de carbone; qui comprend:

(a) la réaction d'un 2-aminonicotinonitrile de formule

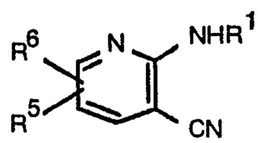

dans laquelle $R^1$, $R^5$ et $R^6$ ont la définition donnée dans la revendication 1, avec (i) un halogénure de (alkyle inférieur)-malonyle, suivie d'une cyclisation dans des conditions basiques, ou (ii) un sel de métal alcalin de malonate de di(alkyle inférieur) dans lequel le terme "inférieur" a la même signification que ci-dessus pour obtenir après acidification un composé de formule I dans laquelle $R^2$ est le groupe $CO_2R^7$ et $R^3$ et $R^4$ représentent chacun de l'hydrogène, ou

(b) la réaction d'un composé de formule

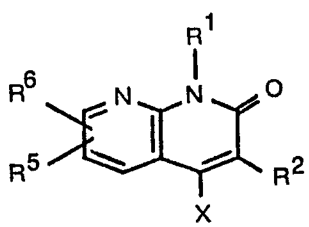

IV

dans laquelle $R^1$, $R^5$ et $R^6$ ont la définition donnée ci-dessus et $R^2$ est un groupe —CN, $COOR^7$ ou —$CONR^8R^9$ où $R^7$, $R^8$ et $R^9$ ont les définitions données ci-dessus et X est le chlore, le brome ou l'iode, avec une amine de formule $HNR^3R^4$ dans laquelle $R^3$ et $R^4$ ont la définition donnée ci-dessus, pour former un composé correspondant de formule I; ou

(c) la réaction d'un composé de formule

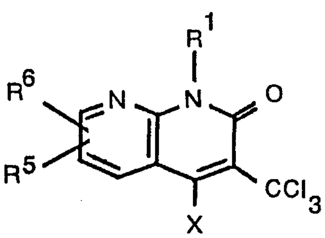

dans laquelle $R^1$, $R^5$ et $R^6$ ont la définition donnée ci-dessus, avec l'ammoniac pour obtenir un composé de formule I, dans laquelle $R^3$ et $R^4$ sont tous deux de l'hydrogène et $R^2$ est le groupe CN ou —$CONH_2$ ou

(d) l'alkylation, l'alcénylation ou l'alcynylation d'un sel de métal alcalin d'un composé de formule I dans laquelle $R^1$ est un métal alcalin pour obtenir un composé de formule I dans laquelle $R^1$ est un groupe alkyle, alcényle ou alcynyle comme défini ci-dessus ou

(e) la transformation d'un ester de formule I dans laquelle $R^2$ est un groupe —$COOR^7$ comme défini ci-dessus pour obtenir un acide de formule I dans laquelle $R^2$ est le groupe COOH, ou un sel pharmaceutiquement acceptable de ce composé, ou

(f) la transformation d'un composé de formule I dans laquelle $R^5$ et/ou $R^6$ représentent un halogène, en un composé de formule I dans laquelle $R^5$ et/ou $R^6$ représentent un groupe alkoxy inférieur ayant 1 à 6 atomes de carbone ou un groupe (alkyle inférieur)amino ayant 1 à 4 atomes de carbone avec

transformation simultanée de $R^2$, lorsqu'il s'agit de $COOR^7$, en l'alkylamide inférieur correspondant, ou

(g) la réaction d'un composé de formule I dans laquelle $R^2$ est un groupe $CO_2R^7$ avec l'hydrazine, l'ammoniac ou une amine de formule $HNR^8R^9$ dans laquelle $R^8$ et $R^9$ ont la définition donnée ci-dessus pour obtenir un composé de formule I dans laquelle $R^2$ est un groupe $—CONHNH_2$, $—CONH_2$ ou $—CONR^8R^9$, ou

(h) l'acylation d'un composé de formule I dans laquelle $R^3$ et $R^4$ sont tous deux de l'hydrogène pour obtenir un composé de formule I dans laquelle $R^3$ et/ou $R^4$ sont des groupes alcanoyle ayant 2 à 4 atomes de carbone ou halogénalcanoyle ayant 2 à 4 atomes de carbone, ou

(i) la transformation d'un composé de formule I comme défini ci-dessus en un sel pharmaceutiquement acceptable.

2. Procédé suivant la revendication 1, dans lequel $R^1$ et $R^7$, dans le produit, sont des groupes alkyle ayant 1 à 4 atomes de carbone.

3. Procédé suivant la revendication 1 ou la revendication 2, dans lequel $R^3$ et $R^4$ dans le produit sont tous deux de l'hydrogène ou forment conjointement avec l'atome d'azote auquel ils sont liés un noyau pyrrolidinyle, morpholinyle, pipérazinyle ou 4-méthylpipérazinyle.

4. Procédé suivant l'une quelconque des revendications 1 à 3, dans lequel $R^5$ est l'hydrogène et $R^6$ est l'hydrogène ou le groupe méthyle dans le produit.

5. Procédé suivant la revendication 1, dans lequel le produit est un composé de formule:

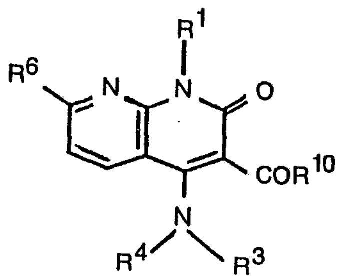

II

dans laquelle

$R^1$ est l'hydrogène ou le groupe méthyle, éthyle, propyle, allyle, propargyle, cyclohexyle ou isobutyle;

$R^3$ est l'hydrogène, un groupe alkyle ayant 1 à 6 atomes de carbone ou le groupe trifluoracétyle;

$R^4$ est l'hydrogène, un groupe alkyle ayant 1 à 6 atomes de carbone, ou forme avec $R^3$ et l'atome d'azote auquel ils sont liés, un noyau pyrrolidinyle, morpholinyle, pipérazinyle ou 4-méthylpipérazinyle;

$R^6$ est l'hydrogène ou le groupe méthyle;

$R^{10}$ est un groupe hydroxy, alkoxy ayant 1 à 6 atomes de carbone ou hydrazinyle; ou un sel pharmaceutiquement acceptable de ce composé.

6. Procédé suivant la revendication 1 pour la préparation d'une 4-amino-1-alkyl-1,2-dihydro-2-oxo-1,8-naphtyridine, dans lequel le produit est:

l'ester éthylique de l'acide 4-amino-7-chloro-1-éthyl-1,2-dihydro-2-oxo-1,8-naphtyridine-3-carboxylique, ou

l'ester éthylique de l'acide 4-amino-7-éthoxy-1-éthyl-1,2-dihydro-2-oxo-1,8-naphtyridine-3-carboxylique, ou

le 4-amino-7-éthylamino-1,N-diéthyl-1,2-dihydro-2-oxo-1,8-naphtyridine-3-carboxamide, ou

l'ester éthylique de l'acide 4-amino-1-éthyl-1,2-dihydro-7-méthyl-2-oxo-1,8-naphtyridine-3-carboxylique, ou

le 4-amino-1-éthyl-1,2-dihydro-7-méthyl-2-oxo-1,8-naphtyridine-3-carbonitrile, ou

le 4-amino-1-éthyl-1,2-dihydro-7-méthyl-2-oxo-1,8-naphtyridine-3-carboxamide, ou

l'ester éthylique de l'acide 4-amino-1-méthyl-1,2-dihydro-2-oxo-1,8-naphtyridine-3-carboxylique, ou

l'ester n-butylique de l'acide 4-amino-1-éthyl-1,2-dihydro-2-oxo-1,8-naphtyridine-3-carboxylique, ou

l'acide 4-amino-1-éthyl-1,2-dihydro-2-oxo-1,8-naphtyridine-3-carboxylique, ou

l'ester éthylique de l'acide 4-amino-1-propyl-1,2-dihydro-2-oxo-1,8-naphtyridine-3-carboxylique, ou

l'ester éthylique de l'acide 4-amino-1-isobutyl-1,2-dihydro-2-oxo-1,8-naphtyridine-3-carboxylique, ou

l'ester éthylique de l'acide 4-amino-1-cyclohexyl-1,2-dihydro-2-oxo-1,8-naphtyridine-3-carboxylique; ou un sel pharmaceutiquement acceptable de ce composé.

7. Procédé suivant la revendication 1 pour la préparation d'une 4-amino-1,2-dihydro-2-oxo-1,8-naphtyridine, dans lequel le produit est:

l'ester éthylique de l'acide 4-amino-1,2-dihydro-2-oxo-1,8-naphtyridine-3-carboxylique, ou

l'acide 4-amino-1,2-dihydro-2-oxo-1,8-naphtyridine-3-carboxylique; ou un sel pharmaceutiquement acceptable de ce composé.

8. Procédé suivant la revendication 1, dans lequel le produit est:
l'ester éthylique de l'acide 1-allyl-4-amino-1,2-dihydro-2-oxo-1,8-naphtyridine-3-carboxylique ou un sel pharmaceutiquement acceptable de ce composé.

9. Procédé suivant la revendication 1, dans lequel le produit est:
l'ester éthylique de l'acide 1-propargyl-4-amino-2-oxo-1,8-naphtyridine-3-carboxylique ou un sel pharmaceutiquement acceptable de ce composé.

10. Procédé suivant la revendication 1, pour la préparation d'une 4-pyrrolidinyl-1-alkyl-1,2-dihydro-2-oxo-1,8-naphtyridine, dans lequel le produit est:
l'ester éthylique de l'acide 1-méthyl-4-(1-pyrrolidinyl)-1,2-dihydro-2-oxo-1,8-naphtyridine-3-carboxylique, ou
l'ester éthylique de l'acide 1,7-diméthyl-4-(1-pyrrolidinyl)-1,2-dihydro-2-oxo-1,8-naphtyridine-3-carboxylique, ou
le 1-éthyl-1,2-dihydro-7-méthyl-2-oxo-4-(1-pyrrolidinyl)-1,8-naphtyridine-3-carboxamide, ou
l'ester éthylique de l'acide 1-éthyl-1,2-dihydro-7-méthyl-2-oxo-4-(1-pyrrolidinyl)-1,8-naphtyridine-3-carboxylique; ou un sel pharmaceutiquement acceptable de ce composé.

11. Procédé suivant la revendication 1, dans lequel le produit est:
l'ester éthylique de lacide 1-allyl-1,2-dihydro-7-méthyl-2-oxo-4-(1-pyrrolidinyl)-1,8-naphtyridine-3-carboxylique ou un sel pharmaceutiquement acceptable de ce composé.

12. Procédé suivant la revendication 1, pour la préparation d'une 4-(4-pipérazinyl substitué)-1,2-dihydro-2-oxo-1,8-naphtyridine dans lequel le produit est:
l'ester éthylique de l'acide 1-méthyl-4-(4-méthyl-1-pipérazinyl)-1,2-dihydro-2-oxo-1,8-naphtyridine-3-carboxylique, ou
l'ester éthylique de l'acide 1,7-diméthyl-4-(4-méthyl-1-pipérazinyl)-1,2-dihydro-2-oxo-1,8-naphtyridine-3-carboxylique, ou
l'ester éthylique de l'acide 1-éthyl-1,2-dihydro-4-(4-méthyl-1-pipérazinyl)-2-oxo-1,8-naphtyridine-3-carboxylique, ou
l'ester éthylique de l'acide 1-éthyl-1,2-dihydro-7-méthyl-2-oxo-4-(4-phényl-1-pipérazinyl)-1,8-naphtyridine-3-carboxylique, ou
l'ester diéthylique de l'acide 4,4'-(1,4-pipérazinediyl)-bis[1-éthyl-1,2-dihydro-7-méthyl-2-oxo-1,8-naphtyridine-3-carboxylique], ou
l'ester éthylique de l'acide 1-éthyl-1,2-dihydro-4-(4-carbéthoxy-1-pipérazinyl)-7-méthyl-2-oxo-1,8-naphtyridine-3-carboxylique, ou un sel pharmaceutiquement acceptable de ce composé.

13. Procédé suivant la revendication 1, pour la préparation d'une 4-alkyl- ou -(alkyle substitué)-amino-1,2-dihydro-2-oxo-1,8-naphtyridine dans lequel le produit est:
l'ester éthylique de l'acide 4-diéthylamino-1-éthyl-1,2-dihydro-7-méthyl-2-oxo-1,8-naphtyridine-3-carboxylique, ou
l'ester éthylique de l'acide 1-éthyl-1,2-dihydro-4-éthyl-amino-7-méthyl-2-oxo-1,8-naphtyridine-3-carboxylique, ou
l'ester éthylique de l'acide 1-éthyl-1,2-dihydro-4-[2-(hydroxyéthyl)méthylamino]-7-méthyl-2-oxo-1,8-naphtyridine-3-carboxylique, ou
l'ester éthylique de l'acide 1-éthyl-1,2-dihydro-4-(3-diméthylaminopropylamino)-7-méthyl-2-oxo-1,8-naphtyridine-3-carboxylique; ou un sel pharmaceutiquement acceptable de ce composé.

14. Procédé suivant la revendication 1 pour la préparation d'une 4-acylamino-1,2-dihydro-2-oxo-1,8-naphtyridine dans lequel le produit est:
l'ester éthylique de l'acide 4-diacétylamino-1-éthyl-1,2-dihydro-2-oxo-1,8-naphtyridine-3-carboxylique, ou
l'ester éthylique de l'acide 1-éthyl-1,2-dihydro-4-trifluoracétylamino-2-oxo-1,8-naphtyridine-3-carboxylique, ou
l'acide 1-allyl-4-chloroacétylamino-1,2-dihydro-2-oxo-1,8-naphtyridine-3-carboxylique; ou un sel pharmaceutiquement acceptable de ce composé.

15. Procédé suivant la revendication 1, dans lequel le produit est:
l'acide 1-éthyl-1,2-dihydro-7-méthyl-4-(4-morpholinyl)-2-oxo-1,8-naphtyridine-3-carboxylique, ou un sel pharmaceutiquement acceptable de ce composé.

16. Procédé suivant la revendication 1 pour la préparation d'une 4-amino-1-éthyl-1,2-dihydro-2-oxo-1,8-naphtyridine, dans lequel le produit est:
l'ester éthylique de l'acide 4-amino-1-éthyl-1,2-dihydro-2-oxo-1,8-naphtyridine-3-carboxylique, ou
l'hydrazide de l'acide 4-amino-1-éthyl-1,2-dihydro-2-oxo-1,8-naphtyridine-3-carboxylique, ou un sel pharmaceutiquement acceptable de ce composé.

17. Procédé suivant la revendication 1 pour la préparation d'une 4-pipérazinyl-1-éthyl-1,2-dihydro-2-oxo-1,8-naphtyridine dans lequel le produit est:
l'acide 1-éthyl-1,2-dihydro-7-méthyl-2-oxo-4-(4-méthyl-1-pipérazinyl)-1,8-naphtyridine-3-carboxylique ou l'ester éthylique de l'acide 1-éthyl-1,2-dihydro-7-méthyl-4-(4-méthyl-1-pipérazinyl)-2-oxo-1,8-naphtyridine-3-carboxylique ou l'ester éthylique de l'acide 1-éthyl-1,2-dihydro-7-méthyl-2-oxo-4-(1-pipérazinyl)-1,8-naphtyridine-3-carboxylique ou un sel pharmaceutiquement acceptable de ce composé.

18. Procédé de production d'une composition thérapeutique, caractérisé en ce qu'un composé de formule I comme défini dans la revendication 1 ou un sel pharmaceutiquement acceptable de ce composé et un support pharmaceutiquement acceptable sont mis sous une forme qui convient à l'administration thérapeutique.

**Patentansprüche für die Vertragsstaaten: BE CH DE FR IT LU NL LI SE**

1. Eine Verbindung der Formel

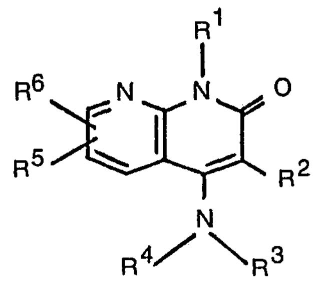

, (I)

worin $R^1$ Wasserstoff, Alkyl mit 1 bis 6 C-Atomen, Cyclo-alkyl mit 5 bis 6 C-Atomen, Alken-(3,4,5 oder 6)-yl mit 3 bis 6 C-Atomen oder Alkin-(3,4,5 oder 6)-yl mit 3 bis 6 C-Atomen bedeutet; $R^2$ —CH, —$CO_2H$, —$CO_2R^7$, wobei $R^7$ Alkyl mit 1 bis 6 C-Atomen ist, —$CONHNH_2$ oder

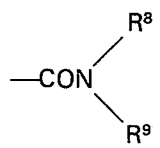

ist, wobei $R^8$ und $R^9$ unabhängig Wasserstoff oder Alkyl mit 1 bis 6 C-Atomen sind, darstellt; $R^3$ und $R^4$ unabhängig Wasserstoff, Alkyl mit 1 bis 6 C-Atomen, Hydroxyalkyl mit 1 bis 6 C-Atomen, Dialkylaminoalkyl mit 4 bis 8 C-Atomen, Alkanoyl mit 2 bis 4 C-Atomen, Halogenalkanoyl mit 2 bis 4 C-Atomen sind oder zusammen mit dem Stickstoffatom, an das sie gebunden sind, eine heterocyclische Gruppe ausgewählt unter Aziridinyl, Azetidinyl, Pyrrolidinyl, Piperidinyl, Piperazinyl, 4-nied.Alkylpiperazinyl, 4-Phenylpiperazinyl, 4-(1-Äthyl-1,2-dihydro-7-methyl-2-oxo-1,8-naphthyridin-4-pyl-3-carbonsäure-äthylester)-piperazinyl, 4-Carb(nied.)alkoxypiperazinyl, Morpholinyl, Thiomorpholinyl oder einem ringsubstituierten nied.Alkylanalogon hievon vervollständigen; $R^5$ und $R^6$ unabhängig Wasserstoff, Alkyl mit 1 bis 4 C-Atomen, Halogen, Alkylamino mit 1 bis 4 C-Atomen oder Alkoxy mit 1 bis 6 C-Atomen bedeuten, oder ein pharmazeutisch annehmbares Salz hievon; wobei der Ausdruck "nied." im Zusammenhang mit Alkyl oder Alkoxy bedeutet, daß derartige Gruppen 1 bis 6 C-Atome enthalten.

2. Eine Verbindung der Formel (I), wie in Anspruch 1 beansprucht, worin $R^1$ und $R^7$ Alkyl mit 1 bis 4 C-Atomen sind.

3. Eine Verbindung der Formel (I), wie in Anspruch 1 oder Anspruch 2 beansprucht, worin $R^3$ und $R^4$ beide Wasserstoff sind oder zusammen mit dem Stickstoffatom, an das sie gebunden sind, einen Pyrrolidinyl-, Morpholinyl-, Piperazinyl- oder 4-Methylpiperazinylring bilden.

4. Eine Verbindung der Formel (I), wie in einem der Ansprüche 1 bis 3 beansprucht, worin $R^5$ Wasserstoff und $R^6$ Wasserstoff oder Methyl sind.

5. Eine Verbindung der Formel:

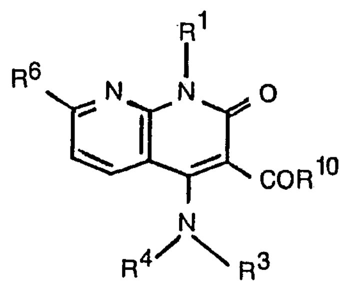

(II)

worin $R^1$ Wasserstoff, Methyl, Äthyl, Propyl, Allyl, Propargyl, Cyclohexyl oder Isobutyl ist;

$R^3$ Wasserstoff, Alkyl mit 1 bis 6 C-Atomen oder Trifluoracetyl bedeutet;

$R^4$ Wasserstoff, Alkyl mit 1 bis 6 C-Atomen ist oder zusammen mit $R^3$ und dem Stickstoffatom, an das sie gebunden sind, einen Pyrrolidinyl-, Morpholinyl-, Piperazinyl- oder 4-Methylpiperazinylring bildet;

$R^6$ Wasserstoff oder Methyl darstellt; und

$R^{10}$ Hydroxy, Alkoxy mit 1 bis 6 C-Atomen oder Hydrazinyl bedeutet; oder ein pharmazeutisch annehmbares Salz hievon.

6. Ein 4-Amino-1-alkyl-1,2-dihydro-2-oxo-1,8-naphthyridin, wie in Anspruch 1 beansprucht, welches ist:
4-Amino-7-chlor-1-äthyl-1,2-dihydro-2-oxo-1,8-naphthyridin-3-carbonsäure-äthylester oder
4-Amino-7-äthoxy-1-äthyl-1,2-dihydro-2-oxo-1,8-naphthyridin-3-carbonsäure-äthylester oder
4-Amino-7-äthylamino-1,N-diäthyl-1,2-dihydro-2-oxo-1,8-naphthyridin-3-carboxamid oder
4-Amino-1-äthyl-1,2-dihydro-7-methyl-2-oxo-1,8-naphthyridin-3-carbonsäure-äthylester oder
4-Amino-1-äthyl-1,2-dihydro-7-methyl-2-oxo-1,8-naphthyridin-3-carbonitril oder
4-Amino-1-äthyl-1,2-dihydro-7-methyl-2-oxo-1,8-naphthyridin-3-carboxamid oder
4-Amino-1-methyl-1,2-dihydro-2-oxo-1,8-naphthyridin-3-carbonsäure-äthylester oder
4-Amino-1-äthyl-1,2-dihydro-2-oxo-1,8-naphthyridin-3-carbonsäure-n-butylester oder
4-Amino-1-äthyl-1,2-dihydro-2-oxo-1,8-naphthyridin-3-carbonsäure oder
4-Amino-1-propyl-1,2-dihydro-2-oxo-1,8-naphthyridin-3-carbonsäure-äthylester oder
4-Amino-1-isobutyl-1,2-dihydro-2-oxo-1,8-naphthyridin-3-carbonsäure-äthylester oder
4-Amino-1-cyclohexyl-1,2-dihydro-2-oxo-1,8-naphthyridin-3-carbonsäure-äthylester oder
ein pharmazeutisch annehmbares Salz hievon.

7. Ein 4-Amino-1,2-dihydro-2-oxo-1,8-naphthyridin, wie in Anspruch 1 beansprucht, welches ist:
4-Amino-1,2-dihydro-2-oxo-1,8-naphthyridin-3-carbonsäure-äthylester oder
4-Amino-1,2-dihydro-2-oxo-1,8-naphthyridin-3-carbonsäure oder ein pharmazeutisch annehmbares Salz hievon.

8. 1-Allyl-4-amino-1,2-dihydro-2-oxo-1,8-naphthyridin-3-carbonsäureäthylester oder ein pharmazeutisch annehmbares Salz hievon.

9. 1-Propargyl-4-amino-2-oxo-1,8-naphthyridin-3-carbonsäure-äthylester oder ein pharmazeutisch annehmbares Salz hievon.

10. Ein 4-Pyrroldinyl-1-alkyl-1,2-dihydro-2-oxo-1,8-naphthyridin, wie in Anspruch 1 beansprucht, welches ist:
1-Methyl-4-(1-pyrrolidinyl)-1,2-dihydro-2-oxo-1,8-naphthyridin-3-carbonsäure-äthylester oder
1,7-Dimethyl-4-(1-pyrrolidinyl)-1,2-dihydro-2-oxo-1,8-naphthyridin-3-carbonsäure-äthylester oder
1-Äthyl-1,2-dihydro-7-methyl-2-oxo-4-(1-pyrrolidinyl)-1,8-naphthyridin-3-carboxamid oder
1-Äthyl-1,2-dihydro-7-methyl-2-oxo-4-(1-pyrrolidinyl)-1,8-naphthyridin-3-carbonsaure-äthylester oder
ein pharmazeutisch annehmbares Salz hievon.

11. 1-Allyl-1,2-dihydro-7-methyl-2-oxo-4-(1-pyrrolidinyl)-1,8-naphthyridin-3-carbonsäureäthylester oder ein pharmazeutisch annehmbares Salz hievon.

12. Ein 4-(4-substituiertes Piperazinyl)-1,2-dihydro-2-oxo-1,8-naphthyridin, wie in Anspruch 1 beansprucht, welches 1-
1-Methyl-4-(4-methyl-1-piperazinyl)-1,2-dihydro-2-oxo-1,8-naphthyridin-3-carbonsäure-äthylester oder
1,7-Dimethyl-4-(4-methyl-1-piperazinyl)-1,2-dihydro-2-oxo-1,8-naphthyridin-3-carbonsäure-äthylester oder
1-Äthyl-1,2-dihydro-4-(4-methyl-1-piperazinyl)-2-oxo-1,8-naphthyridin-3-carbonsäure-äthylester oder
1-Äthyl-1,2-dihydro-7-methyl-2-oxo-4-(4-phenyl-1-piperazinyl)-1,8-naphthyridin-3-carbonsäure-äthylester oder
4,4′-(1,4-Piperazindiyl)-bis-(1-äthyl-1,2-dihydro-7-methyl-2-oxo-1,8-naphthyridin-3-carbonsäure)-diäthylester oder
1-Äthyl-1,2-dihydro-4-(4-carbäthoxy-1-piperazinyl)-7-methyl-2-oxo-1,8-naphthyridin-3-carbonsäure-äthylester oder
ein pharmazeutisch annehmbares Salz hievon ist.

13. Ein 4-Alkyl- oder substituiertes Alkyl-amino-1,2-dihydro-2-oxo-1,8-naphthyridin, wie in Anspruch 1 beansprucht, welches ist:
4-Diäthylamino-1-äthyl-1,2-dihydro-7-methyl-2-oxo-1,8-naphthyridin-3-carbonsäure-äthylester oder
1-Äthyl-1,2-dihydro-4-äthylamino-7-methyl-2-oxo-1,8-naphthyridin-3-carbonsäure-äthylester oder
1-Äthyl-1,2-dihydro-4-[2-(hydroxyäthyl)-methylamino]-7-methyl-2-oxo-1,8-naphthyridin-3-carbonsäure-äthylester oder

1-Äthyl-1,2-dihydro-4-(3-dimethylaminopropylamino)-7-methyl-2-oxo-1,8-naphthyridin-3-carbonsäure-äthylester oder

ein pharmazeutisch annehmbares Salz hievon.

14. Ein 4-Acylamino-1,2-dihydro-2-oxo-1,8-naphthyridin, wie in Anspruch 1 beansprucht, welches ist:

4-Diacetylamino-1-äthyl-1,2-dihydro-2-oxo-1,8-naphthyridin-3-carbonsäure-äthylester oder

1-Äthyl-1,2-dihydro-4-trifluoracetylamino-2-oxo-1,8-naphthyridin-3-carbonsäure-äthylester oder

1-Allyl-4-chloracetylamino-1,2-dihydro-2-oxo-1,8-naphthyridin-3-carbonsäure-äthylester oder

ein pharmazeutisch annehmbares Salz hievon.

15. 1-Äthyl-1,2-dihydro-7-methyl-4-(4-morpholinyl)-2-oxo-1,8-naphthyridin-3-carbonsäure oder ein pharmazeutisch annehmbares Salz hievon.

16. Ein 4-Amino-1-äthyl-1,2-dihydro-2-oxo-1,8-naphthyridin, wie in Anspruch 1 beansprucht, welches ist:

4-Amino-1-äthyl-1,2-dihydro-2-oxo-1,8-naphthyridin-3-carbonsäure-äthylester oder

4-Amino-1-äthyl-1,2-dihydro-2-oxo-1,8-naphthyridin-3-carbonsäure-hydrazid oder

ein pharmazeutisch annehmbares Salz hievon.

17. Ein 4-Piperazinyl-1-äthyl-1,2-dihydro-2-oxo-1,8-naphthyridin, wie in Anspruch 1 beansprucht, welches ist:

1-Äthyl-1,2-dihydro-7-methyl-2-oxo-4-(4-methyl-1-piperazinyl)-1,8-naphthyridin-3-carbonsäure oder

1-Äthyl-1,2-dihydro-7-methyl-4-(4-methyl-1-piperazinyl)-2-oxo-1,8-naphthyridin-3-carbonsäure-äthylester oder

1-Äthyl-1,2-dihydro-7-methyl-2-oxo-4-(1-piperazinyl)-1,8-naphthyridin-3-carbonsäure-äthylester oder

ein pharmazeutisch annehmbares Salz hievon.

18. Ein Verfahren zum Herstellen einer Verbindung, wie in Anspruch 1 beansprucht, welches umfaßt:

(a) die Umsetzung eines 2-Aminonikotinnitrils der Formel

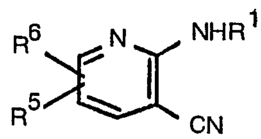

, (V)

worin $R^1$, $R^5$ und $R^6$ wie in Anspruch 1 definiert sind, mit (i) einem nied. Alkylamlonylhalogenid und nachfolgenden Ringschluß unter basischen Bedingungen oder (ii) einem Alkalimetall-di(nied.)-alkylmoalonatester, wobei bei Ansäuerung eine Verbindung der Formel (I) erhalten wird, worin $R^2$ —$CO_2R^7$ ist und $R^3$ und $R^4$ beide Wasserstoff darstellen, wobei der in Verbindung mit Alkyl verwendete Ausdruck "nied." bedeutet, daß eine derartige Gruppe 1 bis 6 C-Atome enthält; oder

(b) die Umsetzung einer Verbindung der Formel

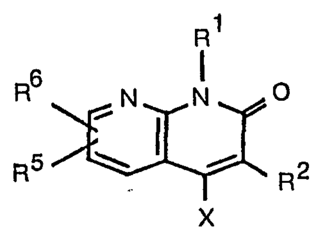

, (IV)

worin $R^1$, $R^5$ und $R^6$ wie in Anspruch 1 definiert sind und $R^2$ —CN, $COOR^7$ oder —$CONR^8R^9$ bedeutet, wobei $R^7$, $R^8$ und $R^9$ wie in Anspruch 1 definiert sind und X Chlor, Brom oder Jod ist, mit einem Amin der Formel $HNR^3R^4$, worin $R^3$ und $R^4$ wie in Anspruch 1 definiert sind, wobei eine entsprechende Verbindung der Formel (I) erhalten wird, oder

(c) die Umsetzung einer Verbindung der Formel

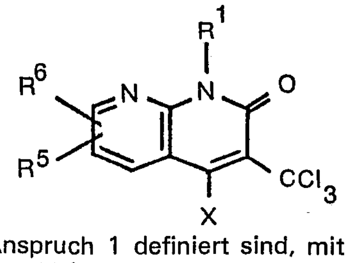

worin X, $R^1$, $R^5$ und $R^6$ wie in Anspruch 1 definiert sind, mit Ammoniak, wobei ein Verbindung der Formel (I) erhalten wird, worin $R^3$ und $R^4$ beide Wasserstoff bedeuten und $R^2$ CN oder —$CONH_2$ ist, oder

(d) Alkylierung, Alkenylierung oder Alkinylierung eines Alkalimetallsalzes einer Verbindung der Formel (I), worin $R^1$ ein Alkalimetall ist, zu einer Verbindung der Formel (I), worin $R^1$ Alkyl, Alkenyl oder Alkinyl ist, wie in Anspruch 1 definiert, oder

(e) Überführung eines Esters der Formel (I), worin $R^2$ $—COOR^7$ ist, wie in Anspruch 1 definiert, in eine Säure der Formel (I), worin $R^2$ COOH ist, oder ein pharmazeutisch annehmbares Salz hievon oder

(f) Überführung einer Verbindung der Formel (I), worin $R^5$ und/oder $R^6$ Halogen ist, in eine Verbindung der Formel (I), worin $R^5$ und/oder $R^6$ nied.Alkoxy mit 1 bis 6 C-Atomen oder nied.Alkylamino mit 1 bis 4 C-Atomen ist, mit gleichzeitiger Überführung von $R^2$, wenn dies $COOR^7$ ist, in das entsprechende nied.Alkylamid oder

(g) Umsetzung einer Verbindung der Formel (I), worin $R^2$ $CO_2R^7$ ist, mit Hydrazin, Ammoniak oder einem Amin der Formel $HNR^8R^9$, worin $R^8$ und $R^9$ wie in Anspruch 1 definiert sind, zu einer Verbindung der Formel (I), worin $R^2$ $—CONHNH_2$, $—CONH_2$ oder $—CONR^8R^9$ ist, oder

(h) Acylierung einer Verbindung der Formel (I), worin $R^3$ und $R^4$ beide Wasserstoff sind, zu einer Verbindung der Formel (I), worin $R^3$ und/oder $R^4$ Alkanoyl mit 2 bis 4 C-Atomen oder Halogenalkanoyl mit 2 bis 4 C-Atomen sind, oder

(i) Überführung einer Verbindung der Formel (I), wie in Anspruch 1 definiert, in ein pharmazeutisch annehmbares Salz.

19. Eine Verbindung der Formel (I), wie in einem der Ansprüche 1 bis 17 beansprucht, zur Verwendung als Pharmazeutikum.

20. Eine pharmazeutische Zusammensetzung, die eine Verbindung der Formel (I), wie in einem der Ansprüche 1 bis 17 beansprucht und einen pharmazeutisch annehmbaren Träger umfaßt.

**Patentansprüche für den Vertragsstaat: AT**

1. Verfahren zur Herstellung einer Verbindung der Formel:

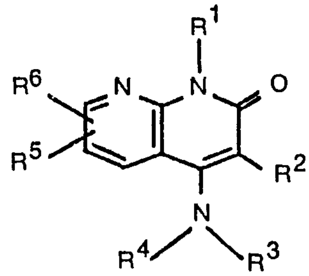

, (I)

worin $R^1$ Wasserstoff, Alkyl mit 1 bis 6 C-Atomen, Cyclo-alkyl mit 5 bis 6 C-Atomen, Alken-(3,4,5 oder 6)-yl mit 3 bis 6 C-Atomen oder Alkin-(3,4,5 oder 6)-yl mit 3 bis 6 C-Atomen bedeutet; $R^2$ —CH, $—CO_2H$, $—CO_2R^7$, wobei $R^7$ Alkyl mit 1 bis 6 C-Atomen ist, $—CONHNH_2$ oder

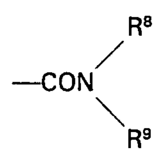

ist, wobei $R^8$ und $R^9$ unabhängig Wasserstoff oder Alkyl mit 1 bis 6 C-Atomen sind, darstellt; $R^3$ und $R^4$ unabhängig Wasserstoff, Alkyl mit 1 bis 6 C-Atomen, Hydroxyalkyl mit 1 bis 6 C-Atomen, Dialkylaminoalkyl mit 4 bis 8 C-Atomen, Alkanoyl mit 2 bis 4 C-Atomen, Halogenalkanoyl mit 2 bis 4 C-Atomen sind oder zusammen mit dem Stickstoffatom, an das sie gebunden sind, eine heterocyclische Gruppe ausgewählt unter Aziridinyl, Azetidinyl, Pyrrolidinyl, Piperidinyl, Piperazinyl, 4-nied.Alkylpiperazinyl, 4-Phenylpiperazinyl, 4-(1-Äthyl-1,2-dihydro - 7 - methyl - 2 - oxo - 1,8 - naphthyridin-4-yl-3-carbonsäure-äthylester)-piperazinyl, 4-Carb(nied.)alkoxypiperazinyl, Morpholinyl, Thiomorpholinyl oder einem ringsubstituierten nied.Alkylanalogen hievon vervollständigen; $R^5$ und $R^6$ unabhängig Wasserstoff, Alkyl mit 1 bis 4 C-Atomen, Halogen, Alkylamino mit 1 bis 4 C-Atomen oder Alkoxy mit 1 bis 6 C-Atomen bedeuten, oder eins pharmazeutisch annehmbaren Salz hievon; wobei der Ausdruck "nied." im Zusammenhang mit Alkyl oder Alkoxy bedeutet, daß derartige Gruppen 1 bis 6 C-Atome enthalten, welches umfaßt:

(a) die Umsetzung eines 2-Aminonikotinnitrils der Formel

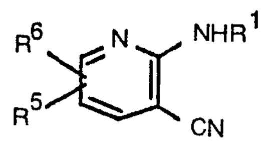

, (V)

worin $R^1$, $R^5$ und $R^6$ wie oben definiert sind, mit (i) einem nied.Alkylmalonylhalogenid und nachfolgenden Ringschluß unter basischen Bedingungen oder (ii) einem Alkalimetall-di(nied.)-alkylmalonatester, wobei bei Ansäuerung eine Verbindung der Formel (I) erhalten wird, worin $R^2$ $—CO_2R^7$ ist und $R^3$ und $R^4$ beide Wasserstoff darstellen, wobei "nied." wie oben definiert ist, oder

(b) die Umsetzung einer Verbindung der Formel

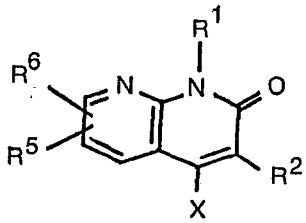

, (IV)

worin $R^1$, $R^5$ und $R^6$ wie oben definiert sind und $R^2$ —CN, $COOR^7$ oder $—CONR^8R^9$ bedeutet, wobei $R^7$, $R^8$ und $R^9$ wie in Anspruch 1 definiert sind und X Chlor, Brom oder Jod ist, mit einem Amin der Formel $HNR^3R^4$, worin $R^3$ und $R^4$ wie oben definiert sind, wobei eine entsprechende Verbindung der Formel (I) erhalten wird, oder

(c) die Umsetzung einer Verbindung der Formel

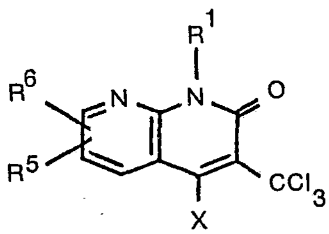

worin X, $R^1$, $R^5$ und $R^6$ wie oben definiert sind, mit Ammoniak, wobei ein Verbindung der Formel (I) erhalten wird, worin $R^3$ und $R^4$ beide Wasserstoff bedeuten und $R^2$ CN oder $—CONH_2$ ist, oder

(d) Alkylierung, Alkenylierung oder Alkinylierung eines Alkalimetallsalzes einer Verbindung der Formel (I), worin $R^1$ ein Alkalimetall ist, zu einer Verbindung der Formel (I), worin $R^1$ Alkyl, Alkenyl oder Alkinyl ist, wie oben definiert, oder

(e) die Überführung eines Esters der Formel (I), worin $R^2$ $—COOR^7$ ist, in eine Säure der Formel (I), worin $R^2$ COOH darstellt, oder ein pharmazeutisch annehmbares Salz hievon, oder

(f) die Überführung einer Verbindung der Formel (I), worin $R^5$ und/oder $R^6$ Halogen bedeuten, in eine Verbindung der Formel (I), worin $R^5$ und/oder $R^6$ nied.Alkoxy mit 1 bis 6 C-Atomen oder nied.Alkylamino mit 1 bis 4 C-Atomen darstellen, mit gleichzeitiger Überführung von $R^2$, wenn $COOR^7$ ist, in das entsprechende nied.Alkylamid, oder

(g) die Umsetzung einer Verbindung der Formel (I), worin $R^2$ $CO_2R^7$ ist, mit Hydrazin, Ammoniak oder einem Amin der Formel $HNR^8R^9$, worin $R^8$ und $R^9$ wie oben definiert sind, wobei eine Verbindung der Formel (I) erhalten wird, worin $R^2$ $—CONHNH_2$, $—CONH_2$ oder $—CONR^8R^9$ bedeutet, oder

(h) die Acylierung einer Verbindung der Formel (I), worin $R^3$ und $R^4$ beide Wasserstoff bedeuten, wobei eine Verbindung der Formel (I) erhalten wird, worin $R^3$ und/oder $R^4$ Alkanoyl mit 2 bis 4 C-Atomen oder Halogenalkanoyl mit 2 bis 4 C-Atomen darstellen, oder

(i) die Überführung einer Verbindung der Formel (I), wie oben definiert, in ein pharmazeutisch annehmbares Salz.

2. Verfahren wie in Anspruch 1 beansprucht, wobei im Produkt $R^1$ und $R^7$ Alkyl mit 1 bis 4 C-Atomen sind.

3. Verfahren wie in Anspruch 1 oder 2 beansprucht, wobei im Produkt $R^3$ und $R^4$ beide Wasserstoff bedeuten oder zusammen mit dem Stickstoffatom, an das sie gebunden sind, einen Pyrrolidinyl-, Morpholinyl-, Piperazinyl- oder 4-Methylpiperazinylring bilden.

4. Verfahren wie in einem der Ansprüche 1 bis 3 beansprucht, wobei im Produkt $R^5$ Wasserstoff und $R^6$ Wasserstoff oder Methyl sind.

5. Verfahren wie in Anspruch 1 beansprucht, wobei das Produkt eine Verbindung der Formel

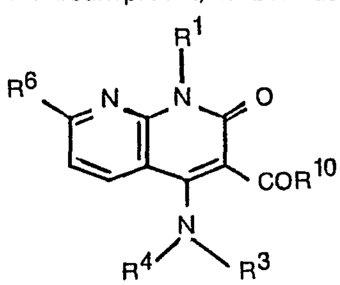

, (II)

worin $R^1$ Wasserstoff, Methyl, Äthyl, Propyl, Allyl, Propargyl, Cyclohexyl oder Isobutyl ist;

$R^3$ Wasserstoff, Alkyl mit 1 bis 6 C-Atomen oder Trifluoracetyl bedeutet;

$R^4$ Wasserstoff, Alkyl mit 1 bis 6 C-Atomen ist oder zusammen mit $R^3$ und dem Stickstoffatom, an das sie gebunden sind, einen Pyrrolidinyl-, Morpholinyl-, Piperazinyl- oder 4-Methylpiperazinylring bildet;

$R^6$ Wasserstoff oder Methyl darstellt; und

$R^{10}$ Hydroxy, Alkoxy mit 1 bis 6 C-Atomen oder Hydrazinyl bedeutet; oder ein pharmazeutisch annehmbares Salz hievon ist.

6. Verfahren wie in Anspruch 1 beansprucht, zur Herstellung eines 4-Amino-1-alkyl-1,2-dihydro-2-oxo-1,8-naphthyridins, in welchem das Produkt ist:

4-Amino-7-chlor-1-äthyl-1,2-dihydro-2-oxo-1,8-naphthyridin-3-carbonsäure-äthylester oder
4-Amino-7-äthoxy-1-äthyl-1,2-dihydro-2-oxo-1,8-naphthyridin-3-carbonsäure-äthylester oder
4-Amino-7-äthylamino-1,N-diäthyl-1,2-dihydro-2-oxo-1,8-naphthyridin-3-carboxamid oder
4-Amino-1-äthyl-1,2-dihydro-7-methyl-2-oxo-1,8-naphthyridin-3-carbonsäure-äthylester oder
4-Amino-1-äthyl-1,2-dihydro-7-methyl-2-oxo-1,8-naphthyridin-3-carbonitril oder
4-Amino-1-äthyl-1,2-dihydro-7-methyl-2-oxo-1,8-naphthyridin-3-carboxamid oder
4-Amino-1-methyl-1,2-dihydro-2-oxo-1,8-naphthyridin-3-carbonsäure-äthylester oder
4-Amino-1-äthyl-1,2-dihydro-2-oxo-1,8-naphthyridin-3-carbonsäure-n-butylester oder
4-Amino-1-äthyl-1,2-dihydro-2-oxo-1,8-naphthyridin-3-carbonsäure oder
4-Amino-1-propyl-1,2-dihydro-2-oxo-1,8-naphthyridin-3-carbonsäure-äthylester oder
4-Amino-1-isobutyl-1,2-dihydro-2-oxo-1,8-naphthyridin-3-carbonsäure-äthylester oder
4-Amino-1-cyclohexyl-1,2-dihydro-2-oxo-1,8-naphthyridin-3-carbonsäure-äthylester oder
ein pharmazeutisch annehmbares Salz hievon.

7. Verfahren wie in Anspruch 1 beansprucht, zur Herstellung eines 4-Amino-1,2-dihydro-2-oxo-1,8-naphthyridin, in welchem das Produkt ist:

4-Amino-1,2-dihydro-2-oxo-1,8-naphthyridin-3-carbonsäure-äthylester oder
4-Amino-1,2-dihydro-2-oxo-1,8-naphthyridin-3-carbonsäure oder ein pharmazeutisch annehmbares Salz hievon.

8. Verfahren wie in Anspruch 1 beansprucht worin das Produkt 1-Allyl-4-amino-1,2-dihydro-2-oxo-1,8-naphthyridin-3-carbonsäure-äthylester oder ein pharmazeutisch annehmbares Salz hievon.

9. Verfahren wie in Anspruch 1 beansprucht, worin das Produkt 1-Propargyl-4-amino-2-oxo-1,8-naphthyridin-3-carbonsäure-äthylester oder ein pharmazeutisch annehmbares Salz hievon ist.

10. Verfahren wie in Anspruch 1 beansprucht, zur Herstellung eines 4-Pyrroldinyl-1-alkyl-1,2-dihydro-2-oxo-1,8-naphthyridins, in welchem das Produkt ist:

1-Methyl-4-(1-pyrrolidinyl)-1,2-dihydro-2-oxo-1,8-naphthyridin-3-carbonsäure-äthylester oder
1,7-Dimethyl-4-(1-pyrrolidinyl)-1,2-dihydro-2-oxo-1,8-naphthyridin-3-carbonsäure-äthylester oder
1-Äthyl-1,2-dihydro-7-methyl-2-oxo-4-(1-pyrrolidinyl)-1,8-naphthyridin-3-carboxamid-oder
1-Äthyl-1,2-dihydro-7-methyl-2-oxo-4-(1-pyrrolidinyl)-1,8-naphthyridin-3-carbonsäure-äthylester oder
ein pharmazeutisch annehmbares Salz hievon.

11. Verfahren wie in Anspruch 1 beansprucht, in welchem das Produkt 1-Allyl-1,2-dihydro-7-methyl-2-oxo-4-(1-pyrrolidinyl)-1,8-naphthyridin-3-carbonsäure-äthylester oder ein pharmazeutisch annehmbares Salz hievon.

12. Verfahren wie in Anspruch 1 beansprucht, zur Herstellung eines 4-(4-substituiertes Piperazinyl)-1,2-dihydro-2-oxo-1,8-naphthyridin, worin das Produkt ist:

1-Methyl-4-(4-methyl-1-piperazinyl)-1,2-dihydro-2-oxo-1,8-naphthyridin-3-carbonsäure-äthylester oder
1,7-Dimethyl-4-(4-methyl-1-piperazinyl)-1,2-dihydro-2-oxo-1,8-naphthyridin-3-carbonsäure-äthylester oder
1-Äthyl-1,2-dihydro-4-(4-methyl-1-piperazinyl)-2-oxo-1,8-naphthyridin-3-carbonsäure-äthylester oder
1-Äthyl-1,2-dihydro-7-methyl-2-oxo-4-(4-phenyl-1-piperazinyl)-1,8-naphthyridin-3-carbonsäure-äthylester oder
4,4′-(1,4-Piperazindiyl)-bis-(1-äthyl-1,2-dihydro-7-methyl-2-oxo-1,8-naphthyridin-3-carbonsäure)-diäthylester oder
1-Äthyl-1,2-dihydro-4-(4-carbäthoxy-1-piperazinyl)-7-methyl-2-oxo-1,8-naphthyridin-3-carbonsäure-äthylester oder
ein pharmazeutisch annehmbares Salz hievon.

13. Verfahren wie in Anspruch 1 beansprucht, zur Herstellung eines 4-Alkyl- oder substituierten Alkyl-amino-1,2-dihydro-2-oxo-1,8-naphthyridins, worin das Produkt ist:

4-Diäthylamino-1-äthyl-1,2-dihydro-7-methyl-2-oxo-1,8-naphthyridin-3-carbonsäure-äthylester oder

1-Äthyl-1,2-dihydro-4-äthylamino-7-methyl-2-oxo-1,8-naphthyridin-3-carbonsäure-äthylester oder

1-Äthyl-1,2-dihydro-4-[2-(hydroxyäthyl)-methylamino]-7-methyl-2-oxo-1,8-naphthyridin-3-carbonsäure-äthylester oder

1-Äthyl-1,2-dihydro-4-(3-dimethylaminopropylamino)-7-methyl-2-oxo-1,8-naphthyridin-3-carbonsäure-äthylester oder

ein pharmazeutisch annehmbares Salz hievon.

14. Verfahren wie in Anspruch 1 beansprucht, zur Herstellung eines 4-Acylamino-1,2-dihydro-2-oxo-1,8-naphthyridins, worin das Produkt ist:

4-Diacetylamino-1-äthyl-1,2-dihydro-2-oxo-1,8-naphthyridin-3-carbonsäure-äthylester oder

1-Äthyl-1,2-dihydro-4-trifluoracetylamino-2-oxo-1,8-naphthyridin-3-carbonsäure-äthylester oder

1-Allyl-4-chloracetylamino-1,2-dihydro-2-oxo-1,8-naphthyridin-3-carbonsäure oder ein pharmazeutisch annehmbares Salz hievon.

15. Verfahren wie in Anspruch 1 beansprucht, worin das Produkt 1-Äthyl-1,2-dihydro-7-methyl-4-(4-morpholinyl)-2-oxo-1,8-naphthyridin-3-carbonsäure oder ein pharmazeutisch annehmbares Salz hievon ist.

16. Verfahren wie in Anspruch 1 beansprucht, zur Herstellung eines 4-Amino-1-äthyl-1,2-dihydro-2-oxo-1,8-naphthyridins, worin das Produkt ist:

4-Amino-1-äthyl-1,2-dihydro-2-oxo-1,8-naphthyridin-3-carbonsäure-äthylester oder

4-Amino-1-äthyl-1,2-dihydro-2-oxo-1,8-naphthyridin-3-carbonsäure-hydrazid oder

ein pharmazeutisch annehmbares Salz hievon.

17. Verfahren wie in Anspruch 1 beansprucht, zur Herstellung eines 4-Piperazinyl-1-äthyl-1,2-dihydro-2-oxo-1,8-naphthyridins, worin das Produkt ist:

1-Äthyl-1,2-dihydro-7-methyl-2-oxo-4-(4-methyl-1-piperazinyl)-1,8-naphthyridin-3-carbonsäure oder

1-Äthyl-1,2-dihydro-7-methyl-4-(4-methyl-1-piperazinyl)-2-oxo-1,8-naphthyridin-3-carbonsäure-äthylester oder

1-Äthy-1,2-dihydro-7-methyl-2-oxo-4-(1-piperazinyl)-1,8-naphthyridin-3-carbonsäure-äthylester oder

ein pharmazeutisch annehmbares Salz hievon.

18. Verfahren zum Herstellen einer therapeutischen Zussamensetzung, dadurch gekennzeichnet, daß eine Verbindung der Formel (I), wie in Anspruch 1 definiert, oder ein pharmazeutisch annehmbares Salz hievon und ein pharmazeutisch annehmbarer Träger in eine Form gebracht werden, die für therapeutische Verabreichung geeignet ist.